# EUROPÄISCHE PATENTSCHRIFT

(11) **EP 1 285 003 B1**
(45) Veröffentlichungstag und Bekanntmachung des Hinweises auf die Patenterteilung: **08.09.2004**
(21) Anmeldenummer: 01925182.6
(22) Anmeldetag: 24.04.2001
(51) Int. Cl.: C07K 17/12, B01D 71/74

(54) **VERWENDUNG VON SEKUNDÄREM ZELLWANDPOLYMER PROKARYONTISCHER MIKROORGANISMEN**
USE OF A SECONDARY CELL WALL POLYMER OF PROCARYOTIC MICROORGANISMS
UTILISATION DE POLYMERES MEMBRANAIRES SECONDAIRES DE MICRO-ORGANISMES PROCARYOTES

(30) Priorität: 26.04.2000 AT 7322000
(43) Veröffentlichungstag der Anmeldung: 26.02.2003
(73) Patentinhaber: NANO-S Biotechnologie GmbH, 1180 Wien (AT)
(72) Erfinder: SLEYTR, Uwe, B., A-1220 Wien (AT); SARA, Margit, A-2230 Gänserndorf (AT); MADER, Christoph, A-1190 Wien (AT); SCHUSTER, Bernhard, A-1210 Wien (AT); UNGER, Frank, M., A-1180 Wien (AT)
(74) Vertreter: Itze, Peter, Dipl.-Ing.
(86) Internationale Anmeldenummer: PCT/AT2001/000122
(87) Internationale Veröffentlichungsnummer: WO 2001/081425

(56) Entgegenhaltungen:
- WO-A-89/09406
- US-A- 4 752 395
- US-A- 4 886 604

## Beschreibung

Die Erfindung bezieht sich auf die Verwendung von sekundärem Zellwandpolymer prokaryontischer Mikroorganismen sowie auf Verbundkörper aus einem Träger und einer Molekülschicht.

Kristalline Zelloberflächenschichten (sogenannte S-Schichten; "S" für surface) zählen zu den häufigsten Oberflächenstrukturen prokaryontischer Organismen (Archaebakterien und Eubakterien). S-Schichten sind aus einer einzigen Protein- oder Glykoproteinspezies (Mr ca. 50.000 bis 200.000) aufgebaut. [Sleytr, U.B., P. Messner, D. Pum and M. Sára. (eds) 1996. Crystalline Bacterial Cell Surface Proteins. In: Molecular Biology Intelligence Unit. Academic Press, R.G. Landes Company, Austin, USA.; Sleytr, U.B., P. Messner, D. Pum and M. Sára. 1999. Crystalline bacterial cell surface layers (S-layers): from supramolecular cell structure to biomimetics and nanotechnology. Angew. Chem. Int. Ed. 38:1034-1054. ;Sleytr, U.B. and T.J. Beveridge. 1999. Bacterial S-layers. Trends Microbiol. 7(6):253-260.] Isolierte S-Schicht (Glyko)Proteine zahlreicher Organismen haben die Fähigkeit zur Selbstorganisation zu monomolekularen kristallinen Gittern in Suspension, auf festen Trägem (z.B. Silizium-Wafern, Polymeren, Metallen), an der Wasser/Luftgrenzfläche sowie gespreiteten Lipidfilmen und Liposomen. Die Poren der S-Schichten sind von regelmäßiger Größe und Morphologie. Permeabilitätsuntersuchungen haben gezeigt, daß S-Schichten scharfe Molekülmassentrenngrenzen im Ultrafiltrationsbereich aufweisen. Diese Eigenschaften haben auch zur Nutzung von S-Schichten für die Herstellung von Ultrafiltrationsmembranen geführt (europäische Patentschrift Nr. 0 154 620 B1).

Umfangreiche Vorversuche haben gezeigt, daß S-Schicht (Glyko)Proteine von zahlreichen Bacillaceae auf gespreiteten Lipidfilmen (z.B. Phospholipide, Tetraetherlipide und anderen amphiphilen Molekülen) rekristallisiert werden können. Dazu werden die Lipide zunächst in einem Trog an der Wasser/-Luftgrenzfläche nach der Langmuir-Technik in Form einer monomolekularen Schicht gespreitet. Für die Rekristallisation von S-Schicht (Glyko)Proteinen an den Lipidfilm kann es von Vorteil sein, daß der gespreitete Lipidfilm zwischen Barrieren auf einen definierten Spreitungsdruck eingestellt wird. Zur Rekristallisation der S-Schicht (Glyko)Proteine auf dem gespreiteten Lipidfilm werden die gelösten S-Schicht(Glyko)Proteine in die Subphase eingebracht, wo sie entweder durch Diffusion oder durch aktive Mischung der Subphase an den gespreiteten Lipidfilm gelangen. Der pH-Wert, die Ionenstärke und die Ionenzusammensetzung der Subphase können dabei einen Einfluß auf das Rekristallisationsverhalten der S-Schicht (Glyko)Proteine haben. [Sleytr, U.B., P. Messner, D. Pum and M. Sära. (eds) 1996. Crystalline Bacterial Cell Surface Proteins. In: Molecular Biology Intelligence Unit. Academic Press, R.G. Landes Company, Austin, USA.; Sleytr, U.B., P. Messner, D. Pum and M. Sára. 1999. Crystalline bacterial cell surface layers (S-layers): from supramolecular cell structure to biomimetics and nanotechnology. Angew. Chem. Int. Ed. 38:1034-1054. ;Sleytr, U.B. and T.J. Beveridge. 1999. Bacterial S-layers. Trends Microbiol. 7(6):253-260.]

Die Rekristallisation in Form einer geschlossenen monomolekularen S-Schicht erfolgt von Nukleationskeimen (Kristallite) aus. Die einzelnen kristallinen S-Schichtdomänen wachsen dabei so lange, bis sie aufeinander treffen und sich zu einer zusammenhängenden Schicht vereinen. Bei der Rekristallisation von S-Schichten können in Abhängigkeit von den gewählten Bedingungen auch Doppelschichten entstehen, wobei die einzelnen Schichten sich mit der Innen- oder Außenseite verbinden können. Vorversuche haben gezeigt, daß S-Schicht-unterstützte Lipidfilme eine wesentlich höhere mechanische Stabilität als "nackte" Lipidfilme aufweisen. Weitere Vorversuche haben gezeigt, daß durch die Rekristallisation einer S-Schicht die Funktionalität von Lipidmembranen nicht beeinträchtigt wird. So werden die spezifische Leitfähigkeit und spezifische Kapazität von Lipidmembranen im Zuge der Ausbildung einer zusammenhängenden assoziierten S-Schicht nicht signifikant verändert Sleytr, U.B., P. Messner, D. Pum and M. Sára. 1999. Crystalline bacterial cell surface layers (S-layers): from supramolecular cell structure to biomimetics and nanotechnology. Angew. Chem. Int. Ed. 38:1034-1054. [Schuster, B., U.B. Sleytr, A. Diederich, G. Bähr, and M. Winterhalter. 1999. Probing the stability of S-layer-supported planar lipid membranes. Eur. Biophys. J. 28:583-590.; Pum, D. and U.B. Sleytr. 1999. The application of bacterial S-layers in molecular nanotechnology. Trends Biotechnol. 17: 8-12.]Weiters lassen sich funktionelle Moleküle sowohl vor als auch nach der Rekristallisation von S-Schichten in die Lipidmembranen einbauen. Diese Funktionalitätsuntersuchungen wurden sowohl mit Hilfe der Voltage-Clamp als auch mit der Black-Lipid Membrane Technik durchgeführt. [Schuster, B., D. Pum and U.B. Sleytr. 1998. Voltage clamp studies on S-layer supported tetraether lipid membranes. Biochim. Biophys. Acta 1369: 51-60.; Schuster, B., D. Pum, O. Braha, H. Bayley and U.B. Sleytr. 1998. Self-assembled α-hemolysin pores in an S-layer-supported lipid bilayer. Biochim. Biophys. Acta 1370: 280-288.] Dabei konnte am Beispiel von α-Hämolysin (α-HL) gezeigt werden, daß sich diese heptameren Poren (aus sieben identen α-HL Molekülen bestehende Transmembranporen) nur dann ausbilden, wenn die porenbildenden Moleküle von der Lipidseite angehoben werden. Aufgrund der im Ultrafiltrationsbereich liegenden molekularen Siebwirkung der S-Schicht können die α-HL-Moleküle jedoch nicht durch das S-Schichtgitter zur Lipidmembran vordringen. Hingegen konnte gezeigt werden, daß sich kleinere Moleküle (z.B. Ionenkanäle wie Valinomycin), von beiden Seiten aus angehoben, in die Lipidmembran einbauen und in der Folge als funktionelle Ionenkanäle mit elektrophysiologischen Methoden gemessen werden können. Die Wechselwirkung zwischen monomolekularen und bimolekularen Lipidfilmen (einschließlich von Mischungen mit anderen amphiphilen Molekülen, wie z.B. Hexadecylamin) und Liposomen mit S-Schicht (Glyko)Proteingittem wurden bisher mit einem breiten Spektrum biophysikalischer Methoden untersucht Hirn, R., B. Schuster, U.B. Sleytr and T.M. Bayerl. 1999. The effect of S-layer protein adsorption and crystallization on the collective motion of a planar lipid bilayer studied by dynamic light scattering. Biophys, J. 77:2066-2074. [Schuster, B., U.B. Sleytr, A. Diederich, G. Bähr, and M. Winterhalter. 1999. Probing the stability of S-layer-supported planar lipid membranes. Eur. Biophys. J. 28:583-590.; Hianik, T., S. Küpcü, U.B. Sleytr, P. Rybár, R. Krivánek and U. Kaatze. 1999. Interaction of crystalline bacterial cell surface proteins with lipid bilayers in liposomes. A sound velocity study. Colloids Surfaces A 147:331-339.; Mader, C., S. Küpcü, M. Sára and U.B. Sleytr. 1999. Stabilizing effect of an S-layer on liposomes towards thermal or mechanical stress. Biochim. Biophys. Acta 1418:106-116.; Györvary, E., B. Wetzer, U.B. Sleytr, A. Sinner, A. Offenhäusser and W. Knoll. 1999. Lateral diffusion of lipids in silane-, dextran- and S-layer-supported mono- and bilayers. Langmuir 15:1337-1347.; Weygand, M., B. Wetzer, D. Pum, U.B. Sleytr, N. Cuvillier, K. Kjaer, P.B. Howes and M. Lösche. 1999. Bacterial S-layer protein coupling to lipids: X-ray reflectivity and grazing incidence diffraction studies. Biophys. J. 76: 458-468.; Wetzer, B., A. Pfandler, E. Györvary, D. Pum, M. Lösche and U.B. Sleytr. 1998. S-layer reconstitution at phospholipid monolayers. Langmuir. 14: 6899-6906.] Dabei zeigte sich, daß durch die Rekristallisation eines S-Schichtgitters die Eigenschaften des Lipidfilmes (z.B. Fluidität) und molekulare Ordnung wesentlich beeinflußt werden können [Györvary, E., B. Wetzer, U.B. Sleytr, A. Sinner, A. Offenhäusser and W. Knoll. 1999. Lateral diffusion of lipids in silane-, dextran- and S-layer-supported mono- and bilayers. Langmuir 15:1337-1347]. S-Schicht-unterstützte Lipidmembranen entsprechen im wesentlichen dem supramolekularen Bauprinzip der Zellhülle jener Archaebakterien (Archaee), die außerhalb der Cytoplasmamembran nur eine S-Schicht als Zellwandkomponente besitzen. Da isolierte S-Schicht (Glyko)Proteine von Archaebakterien aber wesentlich schwerer an Lipidfilme zur Rekristallisation gebracht werden können, werden vorzugsweise S-Schicht (Glyko)Proteine von anderen prokaryontischen Organismen (z.B. Bacillaceae) verwendet. Die hergestellten kompositen S-Schicht Lipidfilme sind somit biomimetische Strukturen, die den Archaebakterien Zellgrenzflächen (cell envelopes) nachempfunden sind, u.zw. ohne notwendigerweise die nativen Bausteine der Archaebakterien verwenden zu müssen.

Aus Arbeiten und Veröffentlichungen der Anmelder ist bekannt, daß bei Grampositiven Bakterien die S-Schicht (Glyko)Proteine an die darunterliegende rigide Zellwandschicht (die sogenannte peptidoglykanhältige Schicht) über sehr spezifische Wechselwirkung gebunden sein können. Diese spezifische Bindung erfolgt offensichtlich häufig zwischen dem S-Schicht Protein und den sogenannten sekundären Zellwandpolysmeren (in der Folge SCWP). Letztere sind kovalent an die Matrix der Peptidoglykanschicht gebunden und können von dieser (z.B. durch eine Behandlung mit HF) abgespalten und in reiner Form gewonnen werden. Aus dem Vergleich der Aminosäuresequenzen verschiedener S-Schicht Proteine wurden Domänen eruiert, die für die Bindung der S-Schicht an die peptidoglykanhältige Zellwandschicht, insbesondere die SCWP-Komponente, verantwortlich sind.

Wie aus Pum, D. and U.B. Sleytr. 1999. The application of bacterial S-layers in molecular nanotechnology. Trends Biotechnol. 17: 8-12. undSleytr, U.B. and M. Sára. 1997. Bacterial and archaeal S-layer proteins: structure-function relationships and their biotechnological applications. Trends Biotechnol. 15: 20-26. bereits bekannt wirkt die S-Schicht stabilisierend auf Lipidmembranen doch betreffen diese Veröffentlichungen Verfahren, bei denen die S-Schicht Proteine in Trögen aus der Subphase an gespreitete Lipidfilme oder an Black-Lipid Membranen rekristallisiert wurden.

Das Prinzip SUM-unterstützter Lipidmembranen könnte auch zu völlig neuen Anwendungsmöglichkeiten für funktionelle Lipidmembranen führen. Es ist wesentlich einfacher, einen Lipidfilm auf eine SUM aufzuziehen (z.B. mit Langmuir Blodgett- oder Langmuir Schäfer-Technik) oder direkt auf dieser zu assemblieren, als zunächst den Lipidfilm durch Spreitung auf einen Trog herzustellen und dann die S-Schicht Proteine aus der Subphase an den Lipidfilm zu rekristallisieren. Die Verwendung von SUMs würde auch die. Entwicklung neuer Testmethoden nach dem Prinzip der Voltage-Clamp Meßtechnik ermöglichen, wie sie etwa beim Screening pharmazeutischer Substanzen auf ihre Wirkung auf membranintegrierte oder -assoziierte Moleküle (z.B. Ionenkanäle, Signalübertragungsmoleküle) benötigt werden.

Weiters ist auch die Voltage-Clamp Technik bekannt, bei welcher eine fein ausgezogene, mit Pufferlösung gefüllte Pipette mit großer Vorsicht einer (funktionellen) Lipidmembran gerade soweit genähert, daß ein meßbarer Kontakt entsteht. In der Folge werden die Transmembranenfunktionen elektrophysiologisch bestimmt (z.B. Messung der Transmembranströme). Diese weit etablierte Methodik läßt sich jedoch nicht für Paralleluntersuchungen, wie sie für das High-Throughput-Screening (Testung von vielen Substanzen in einem Arbeitsschritt) erforderlich sind, anwenden.

Stand des Wissens sind somit Methoden zur Herstellung kompositer S-Schicht-Lipidfilme, wobei die S-Schicht (Glyko)Proteine aus einer Lösung an Lipidfilmen (z.B. Langmuir Filme, Black-Lipid Membranen) in meso- oder makroskopischen Dimensionen zur Rekristallisation gebracht werden.

Der Erfindung liegt unter anderem die Aufgabe zugrunde, neue Wege der Stabilisierung von Lipidfilmen zu erreichen.

Erfindungsgemäß wird diese Aufgabe dadurch gelöst, daß isoliertes, gereinigtes sekundäres Zellwandpolymer zur orientierten monomolekularen Bindung von (funktionellen) Molekülen, vorzugsweise Molekülschichten und/oder zur Anlagerung von funktionellen Molekülen an Molekülen eines Trägers verwendet werden. Dadurch können die spezifischen Wechselwirkungen zwischen einer oder mehrerer Domänen von S-Schicht-Proteinen und sogenannten sekundären Zellwandpolymeren für die Herstellung von supramolekularen Strukturen genutzt werden.

Die Herstellung dieser S-Schicht Membranen ist bereits Gegenstand eines in vielen Staaten erteilten Patentes (s. z.B. europäische Patentschrift 0154620 B1). Im wesentlichen beruht das Verfahren auf der Ablagerung von S-Schicht Fragmenten oder Zellwandfragmenten S-Schicht-tragender Prokaryonten auf porösen Trägern; (vorzugsweise) Mikrofiltrationsmembranen mit offenzelliger, schwammartiger Struktur oder Membranen, die nach der Kemsgurtechnik (z.B. Nuclepore-Membranen) hergestellt wurden. Nach der Ablagerung einer zusammenhängenden S-Schicht Lage (diese kann auch aus mehreren Einzelschichten bestehen), werden die S-Schicht Gitter intra- und intermolekular chemisch vernetzt (vorzugsweise mit Glutaraldehyd). Bei der Verwendung von Glutaraldehyd als chemischer Crosslinker (chemisches Vernetzungsmittel) werden zur Erhöhung der chemischen Stabilität der S-Schicht Membranen die entstandenen Schiffschen Basen anschließend vorzugsweise mit Borhydrid reduziert. Die so hergestellten S-Schicht Membranen werden aufgrund der molekularen Siebwirkung der S-Schichtgitter als Ultrafiltrationsmembranen eingesetzt. In der Folge wird für diese S-Schicht Ultrafiltrationsmembranen die Abkürzung "SUMs" verwendet.

Durch die Verwendung von SUMs als Träger für funktionelle Lipidmembranen (oder Membranen, die aus anderen filmbildenden, z.B. amphiphilen Molekülen, bestehen), lassen sich im Vergleich zu den oben beschriebenen Verfahren zur Rekristallisation von S-Schichten auf gespreiteten Lipidfilmen wesentliche technologische und anwendungstechnische Vorteile erzielen.

Die Aufbringung oder die Erzeugung von (funktionellen) Lipidfilmen auf SUMs kann in sehr unterschiedlicher Weise erfolgen:
1. Eintauchen und/oder Austauchen der SUMs aus einem auf einen Trog gespreiteten Lipidfilm (z.B. Langmuir Blodgett- oder Langmuir Schäfer-Technik). Während im Falle eines gespreiteten Tetraetherlipidfilmes durch ein einfaches Ein- oder Austauchen bereits eine "funktionelle" Lipidmembran hergestellt werden kann, sind beispielsweise bei Phospholipiden zwei Schritte (Ein- und Austauchvorgänge) erforderlich.
2. Herstellung einer chemisch gebundenen Lipidmonoschicht am S-Schichtgitter, wobei die hydrophoben Teile der amphiphilen Moleküle von der SUM wegstehen. Der zweite Lipidmonofilm wird dann entsprechend der Langmuir Blodgett- oder Langmuir Schäfer-Technik (siehe oben) aufgebracht.
3. In den S-Schichtgittern können entsprechende Funktionalitäten (z.B. Biotinilierung, Cysteinreste, Streptavidinbindungsdomäne) eingebaut werden. Die entsprechenden SUM-Oberflächen können auf diese Weise mit Lipidmolekülen oder anderen amphiphilen Molekülen eine spezifische Wechselwirkung eingehen. Auf diese Weise lassen sich komposite S-Schicht/Lipidmembranen herstellen, bei denen der Lipidfilm entsprechend der repetive vorkommenden spezifischen Bindungsstellen an die S-Schicht (Glyko)Proteinmatrix der SUM gebunden ist.
4. Nach der Aufbringung des Lipidfilmes auf die SUM kann gegebenenfalls eine kovalente chemische Vernetzung von Kopfgruppen der Lipidfilme (oder der amphiphilen Moleküle) mit der S-Schicht Matrix erfolgen. Diese Vernetzungsreaktion erfolgt vorzugsweise im Zuge einer Permeation des Vemetzungsmittels durch den porösen Träger der SUM (z.B. durch die Mikrofiltrationsmembran).

Vorteilhafterweise kann die Bindung der sekundären Zellwandpolymere über entsprechende Domänen der funktionellen Moleküle, vorzugsweise Molekülschichten und/oder des Trägers erfolgen, wodurch erreicht wird, daß einerseits eine genaue Lokalisierung und anderseits auch eine genaue Ausrichtung der funktionellen Moleküle bzw. Molekülschichten vorliegt. Dabei kann die Bindung der sekundären Zellwandpolymere an Molekülen, vorzugsweise Molekülschichten oder den Molekülen des Trägers über lektinartige Bindungen erfolgen, was ebenfalls eine genaue Auswahl der Domänen bzw. Epitope zur Bindung der Moleküle bzw. Molekülschichten an den Trägem ermöglicht.

Eine bevorzugte erfindungsgemäßen Verwendung besteht darin, daß die sekundären Zellwandpolymere die Bindeglieder zwischen polymeren Trägerstrukturen und funktionellen Molekülen, Molekülgruppen bzw. Molekülschichten sind, wodurch die polymeren Trägerstrukturen direkt mit entsprechenden funktionellen Molekülen bestückt werden können. Dabei können die Trägerstrukturen Mikrofiltrationsmembranen sein. Weiters können die Molekülschichten in Form von monomolekular kristallin geordneten Proteinschichten vorliegen, wodurch aufgrund der kristallinen Struktur der Proteinschicht einerseits entsprechende Filter mit scharfem Cut-Off erzielt werden können und anderseits diese Proteinmoleküle ihrerseits wieder mit funktionellen Molekülen direkt oder indirekt bestückt sein können. Dazu zählt, daß die monomolekular kristallinen geordneten Proteinschichten ihrerseits Träger einer funktionellen Lipidmembran sind, wodurch sehr stabile Kompositstrukturen erzielt werden, die beispielsweise für Hyperfiltrationen u.dgl. einsetzbar sind. Weiters können die monomolekular geordneten kristallinen Proteinschichten an ihren, den sekundären Zellwandpolymeren abgewandten Seiten mit je einer oder mehreren gleichen oder verschiedenen funktionellen Domänen versehen sein, wodurch die Proteinschichten als Mittler für eine geordnete Bindung von weiteren Molekülen verwendet sind. Für spezielle Bindungsmöglichkeiten können die monomolekular geordneten Moleküle amphiphilen Charakter haben und aus hydrophoben Ketten sowie hydrophilen sekundären Zellwandpolymeren bestehen. Dadurch kann die Richtung der Anlagerung an eine wässerige oder nichtwässerige Phase gesteuert werden. Weiters können die Moleküle des Trägers monomolekular kristallin geordnete Proteinschichten sein, wobei die sekundären Zellwandpolymere unter Bildung eines zusammengesetzten Verbundkörpers Bindeglieder zu einer funktionellen Lipidschicht bilden. Solcherart werden die Lipidmoleküle entsprechend von den Proteinschichten distanziert, wobei zudem ein fester Halt der Lipidschicht an den Proteinschichten erzielt ist. Dabei können über die funktionellen Lipidschichten zwei zusammengesetzte Verbundkörper spiegelbildlich aneinander gebunden sein. Dies ermöglicht die Ausbildung von Schichtungen entsprechend dem sogenannten Sandwich-Prinzip. Dabei können die monomolekular kristallin geordneten Proteinschichten aus gleichen oder unterschiedlichen Proteinmolekülen bestehen, was ermöglicht, daß an den Außenseiten der Verbundkörper unterschiedliche Bindungsdomänen, Ladungen, funktionelle Moleküle u.dgl. vorhanden sind, was das Einsatzgebiet stark erweitert.

Schließlich kann die funktionelle Lipidschicht vesikuläre Struktur aufweisen, was der Natur entsprechend angepaßte künstliche Verbundkörper ermöglicht.

Die Erfindung bezieht sich, wie schon angeführt, auch auf einen Verbundkörper aus einem Träger und einer Molekülschicht, welche erfindungsgemäß dadurch gekennzeichnet ist, daß die Moleküle dieser Molekülschicht orientiert monomolekular an dem Träger entsprechend der erfindungsgemäßen Verwendung über extrahierte, gereinigte sekundäre Zellwandpolymerketten gebunden sind, wobei die Enden der Polymerketten modifiziert sind.Dies ermöglicht die Bildung genau strukturierter chemisch und funktionsmäßig genau definierter Verbundkörper.

Dabei können die sekundären Zellwandpolymerketten am Träger und/oder den zu bindenden Molekülen über eine lektinartige Bindung verankert sein, wodurch eine den passenden Domänen der Proteinmoleküle entsprechende Bindung erzielt ist. Um einen stabilen Verbundkörper zu erhalten, können auf einer Polymermatrix monomolekular kristallin geordnete Proteinschichten über die sekundären Zellwandpolymere gebunden sein, wobei diese sekundären Zellwandpolymere sowohl der Verankerung als auch der Ausrichtung und/oder der gezielten Verteilung dienen. Es können auch an den monomolekular kristallin geordneten Proteinschichten an der den sekundären Zellwandpolymeren abgewandten Seite Lipidfilme oder Vesikel angeordnet sein, wodurch weitere Verwendungsmöglichkeiten gegeben sind. Weiters können an den monomolekular kristallin geordneten Proteinschichten an der den sekundären Zellwandpolymeren abgewandten Seite funktionelle Domänen in regelmäßigen Abständen und Anordnungen vorgesehen sein, was eine gezielten Anlagerung von weiteren funktionellen Molekülen an den Proteinschichten ermöglicht.

Um eine verschiedenartige Bindung bzw. Verteilung von funktionellen Molekülen zu erzielen, können die funktionellen Domänen unterschiedlich, jedoch in regelmäßigen Mustern vorgesehen sein.

Weiters können die sekundären Zellwandpolymere von einer monomolekular kristallin geordneten Proteinschicht, die vorzugsweise direkt auf einen Träger aufgebracht ist, wegragen und eine funktionelle Lipidschicht binden. Damit wird aufgrund der vorzugsweise direkt auf einen Träger aufgebrachten Proteinschicht eine stabile Trägerschicht erhalten, wobei die Lipidschichten frei von Wechselwirkungen zur Proteinschicht an die sekundären Zellwandpolymere gebunden sind. Dabei können zwei derartige Proteinschichten über die funktionelle Lipidschicht spiegelbildlich gegenüberliegend aneinander gebunden sein, wodurch ein stabiler sandwichartig aufgebauter Verbundkörper erzielt ist. Dabei kann die eine Proteinschicht aus anderen Proteinmolekülen bestehen als die andere Proteinschicht, sodaß an den beiden Seiten des Verbundkörpers unterschiedliche Bindungsverhältnisse, Domänen und Funktionen vorliegen können.

In Abwandlung der oben beschriebenen bekannten Voltage-Clamp Technik kann der Verbundkörper die Mündungsöffnung an einem Ende eines Röhrchens überspannend angeordnet sein. Im Vergleich zu den bekannten offenen Pipetten könnte ein solches mit dem erfindungsgemäßen Verbundkörper versehenes Röhrchen (in der Folge SUM-Röhrchen genannt) viel rascher einer gespreiteten funktionellen Lipidmembran genähert werden, ohne den Film zu zerstören. Auf diese Weise ließen sich beispielsweise zahlreiche nebeneinander angeordnete SUM-Röhrchen gleichzeitig für Meßvorgänge an funktionellen Lipidmembranen verwenden. Grundsätzlich könnte an Meßmethoden gedacht werden, bei denen im ersten Schritt sämtliche SUM-Röhrchen mit funktionellen Lipidmembranen beschichtet werden. Dies könnte durch Eintauchen in einen gespreiteten Tetraetherlipidfilm erfolgen, in dem, in einem vorangegangenen Schritt, funktionelle Moleküle eingebaut werden. [Schuster, B., D. Pum and U.B. Sleytr. 1998. Voltage clamp studies on S-layer supported tetraether lipid membranes. Biochim. Biophys. Acta 1369: 51-60]. Im zweiten Schritt würden die SUM-Röhrchen in kleine Gefäße (z.B. ähnlich Mikrotiterplatten) abgesenkt werden, in denen sich verschiedene Substanzen befinden, deren Wirkung auf die membranassoziierten oder -integrierten funktionellen Moleküle geprüft werden soll. Die elektrophysiologischen Messungen würden entsprechend der klassischen Voltage-Clamp Technik erfolgen.

SUMs könnten in Verbindung mit SCWP auch als Träger für funktionelle Lipidmembranen im makroskopischen Bereich dienen. Gegenwärtig gibt es kaum vergleichbare Trägerschichten zur großflächigen Stabilisierung von Lipidmembranen.

Schließlich können die erfindungsgemäßen Verbundkörper als Träger spezieller funktioneller Moleküle für Diagnostika, Drug-Targeting, Drug-Delivery, Kunstviren-Drugscreening, High-Throughput-Screening od.dgl. eingesetzt werden.

Mit der erfindungsgemäßen Verwendung sind auch vesikuläre Strukturen geschaffen werden, z.B. Liposomen bzw.Lipidtropfen (Lipidpartikel), die mit S-Schichten bedeckt sind.

Schließlich kann als Träger auch eine Micelle aus hydrophoben selbstorganisierten Ketten verwendet werden.

In der Zeichnung sind schematisch verschiedene Ausführungsformen der Verbundkörper im Schnitt und wesentlich vergrößertem Maßstab gezeichnet.

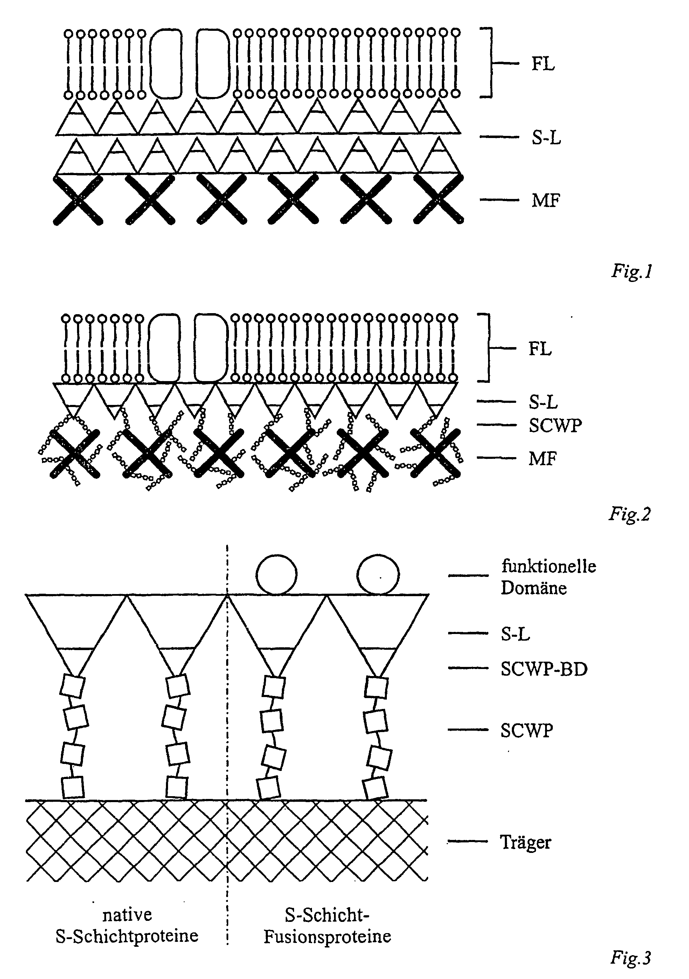

Fig. 1 zeigt die grundsätzliche Nutzung der S-Schichten Ultrafiltrationsmembranen, wobei in allen Abbildungen folgende Abkürzungen verwendet werden:
MF: Mikrofiltrationsmembran,
S-L: S-Schicht (engl. S-Layer),
FL: Funktionelle Lipidmembran, d.h. Lipidmembran mit eingebauter Membranfunktion, insbesondere Transmembranfunktion (z.B. Ionenkanal).

Ein derartiger Aufbau ist bereits Stand der Technik aus den eigenen Arbeiten der Anmelder.

Fig. 2 zeigt die chemische Kopplung des SCWP an ein weiteres Polymer und anschließende Mischung dieser Moleküle mit einem für die Herstellung von Mikrofiltrationsmembranen (mit schwammartiger Struktur) üblichen Polymer. Beim Ausfällungsprozeß in wässrigen Lösungen (z.B. nach dem Phaseninversionsverfahren) sollten die hydrophilen SCWP in die wässrige Phase ragen, während die an ihnen (kovalent gebundenen) Polymerketten in der kompakten Polymerstruktur verankert sind.

Grundsätzlich sollte auch an andere Möglichkeiten gedacht werden, um die Oberflächen von Polymerstrukturen (Folien, schwammartige oder andere poröse Strukturen) mit SCWPs zu funktionalisieren. Bei all diesen Verfahren sollten die hydrophilen SCWP-Ketten in ausreichender Dichte von der Oberfläche der Polymerstrukturen wegragen, um einen Kontakt zu den entsprechenden Bindungsdomänen des S-Schicht Proteins zu ermöglichen.

Neben den Verfahren der Polymerisation können auch chemische Kopplungsmethoden angewendet werden. Beispielsweise könnten an einem Ende des SCWP reaktive Gruppen eingebaut werden, die mit Gruppen an der Oberfläche der Polymerstrukturen reagieren können. Umgekehrt könnten auch voraktivierte Gruppen auf der Polymermatrix mit reaktiven Gruppen auf dem SCWP zur Reaktion gebracht werden.

Entsprechend Fig. 3 könnten dann auf den mit SCWP funktionalisierten Polymeroberflächen S-Schichten aus einer Lösung von S-Schicht (Glyko)Protein rekristallisiert werden. Dieser Vorgang würde zu einer kompletten monomolekularen S-Schicht Bedeckung der Polymeroberfläche führen. Da neben nativen S-Schicht (Glyko)Proteinen auch S-Schicht Fusionsproteine, mit eingebauten spezifischen Funktionen (z.B. Biotin-Bindungsdomäne des Streptavidins, Protein A, Protein G, Antikörper- oder Antigendomänen), für die Rekristallisation verwendet werden können, ließe sich über dieses Verfahren eine sehr einheitliche Funktionalisierung von Polymermatrices erreichen (siehe Fig. 3).

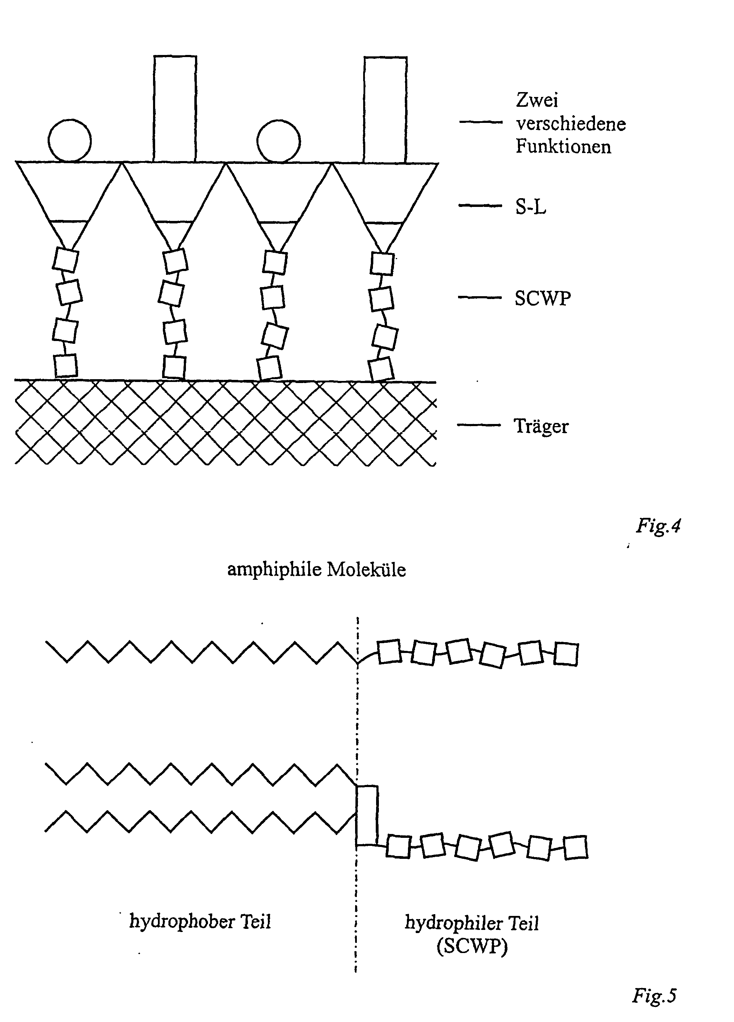

Bei der Verwendung von S-Schicht Fusionsprotein gemäß Fig. 4 könnten gleichzeitig auch zwei oder mehrere idente, aber unterschiedlich funktionalisierte S-Schicht Proteine zum Einsatz kommen. Dadurch würde sich aufgrund der einheitlichen Proteinbasisstrukturen auf den SCWP ein einheitliches S-Schichtgitter ausbilden, das jedoch in diskreten Abständen (aber mit statistischer Verteilung) unterschiedliche Bindungsstellen (funktionelle Domänen) aufweist.

Fig. 5 zeigt die Verwendung von amphiphilen Molekülen, bestehend aus SCWP und hydrophoben Ketten, die im molekularen Strukturkonzept beispielsweise Glykolipiden entsprechen. Ähnliche Moleküle werden beispielsweise auch in den Plasmamembranen von Archaebakterien (Archaea) gefunden. Dabei ist insbesondere die Herstellung der glykolipidäquivalenten Moleküle gezeigt.

Fig. 6 zeigt die Verwendung natürlich vorkommender oder vollsynthetische Moleküle, wobei diese aus einem zentralen hydrophoben Teil und zwei hydrophilen Domänen bestehen.

Fig. 7 veranschaulicht die Nutzung der in Fig. 5 und Fig. 6 dargestellten Moleküle zur Herstellung kompositer SUM/Lipidmembranen. Die Verwendung der in Fig. 5 und 6 schematisch dargestellten amphiphile Moleküle ermöglicht eine breite Variation beim Aufbau kompositer SUM/Lipidmembranen. Besonders hervorzuheben ist die grundsätzliche Möglichkeit, zwei verschiedene SCWP zu verwenden, die an verschiedene S-Schicht Proteine binden.

Fig. 8 illustriert die Herstellung von kompositen S-Schicht/funktionellen Lipidmembranen unter Verwendung von SCWP. In Fig. 8 werden folgende Abkürzungen verwendet:
A: S-Schicht Protein Typ A
SCWP-A: Sekundäres Zellwandpolymer, das spezifisch an S-Schicht Proteine des Typs A bindet,
B: S-Schicht Protein Typ B
SCWP-B: Sekundäres Zellwandpolymer, das spezifisch an S-Schicht Proteine des Typs B bindet.

Auf der Mikrofiltrationsmembran wird zunächst eine S-Schicht (z.B. Typ A) aufgetragen. Dieser Prozeß entspricht im wesentlichen dem bereits oben beschriebenen Verfahren zur Herstellung von SUMs. In der Folge wird auf die SUM eine Monoschicht von amphiphilen Molekülen (geeignet zur Herstellung von funktionellen Lipidmembranen siehe Beispiele) entweder mit Hilfe der Langmuir Schäfer- oder Langmuir Blodgett Technik aufgezogen. Die Anbindung der Monoschicht an die SUM erfolgt durch eine spezifische Wechselwirkung zwischen dem SCWP-Teil der amphiphilen Moleküle und der S-Schicht auf der SUM. Diese Verankerung von Lipidmonoschichten an S-Schichten auf SUMs führte zu einer wesentlichen Erhöhung der Stabilität und Lebensdauer funktioneller Lipidmembranen.

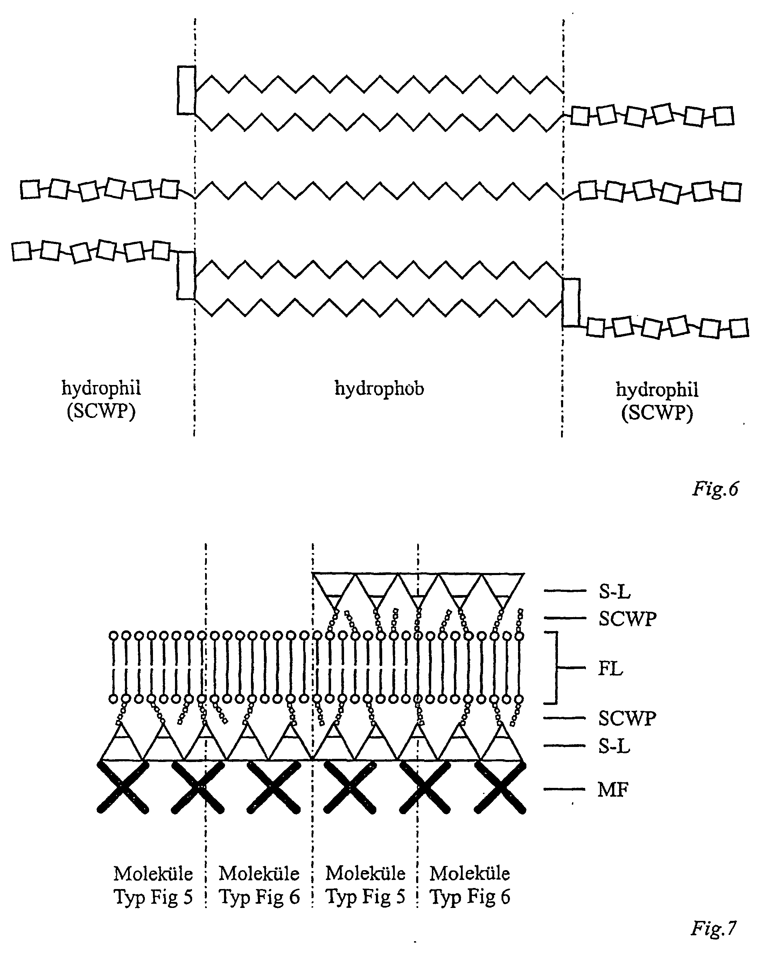

Alternativ zu den in Fig. 5 dargestellten Molekülen können auch die in Fig. 6 angeführten Moleküle zur Herstellung der funktionellen Lipidmembran eingesetzt werden, wobei letztere die Herstellung einer funktionellen Lipidmembran in einem Schritt ermöglichen. Bei Verwendung der in Fig. 5 schematisch dargestellten Moleküle muß hingegen in einem zweiten Schritt eine weitere Monoschicht von amphiphilen Molekülen aufgezogen werden. Enthält diese äußere Schicht amphiphile Moleküle mit einem SCWP-Teil, können die exponierten SCWP-Ketten als spezifische Bindungsstellen für eine weitere S-Schicht dienen. Diese zweite S-Schicht kann beispielsweise auf der Lipidmembran aus einer Lösung von S-Schicht (Glyko)Proteinen rekristallisiert werden. Alternativ dazu kann die S-Schicht auch in einem ersten Schritt auf der Wasser-/Luftgrenze rekristallisiert werden und erst in der Folge auf die Lipidmembran aufgebracht (z.B. abgesenkt) werden.

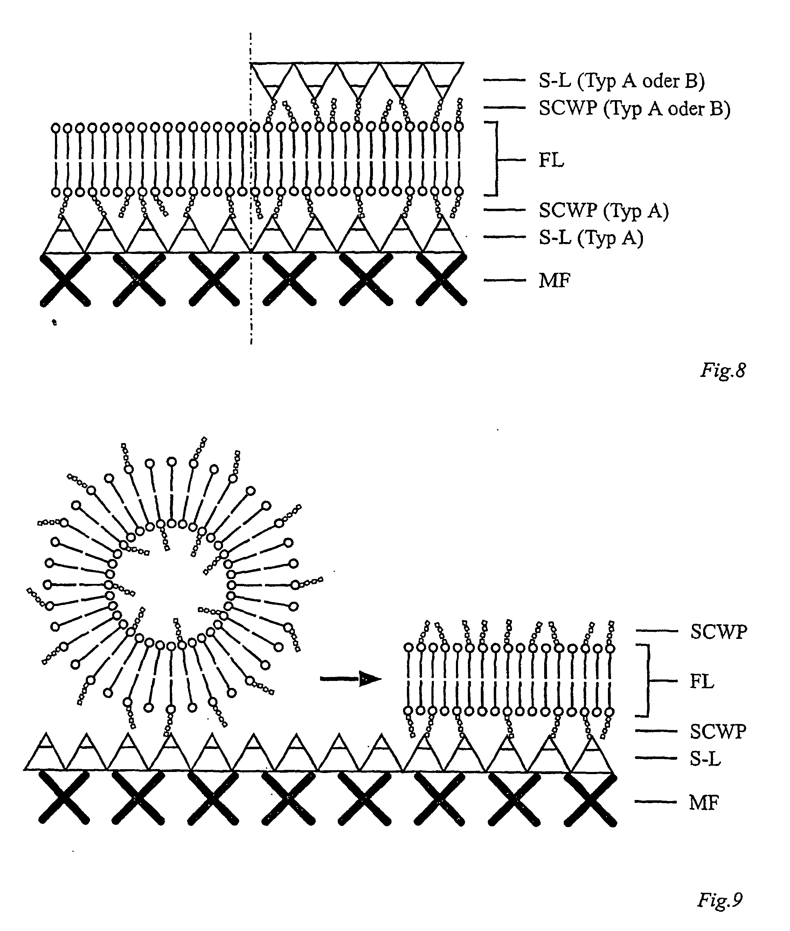

Das in Fig. 8 dargestellte supramolekulare Schichtsystem kann unter Verwendung einheitlicher oder verschiedener SCWP-S-Schicht Partner erfolgen. In der Figur sind Beispiele für S-Schicht Typ A und SCWP Typ A sowie S-Schicht Typ A und SCWP Typ A mit S-Schicht Typ B und SCWP Typ B dargestellt.

Vielfach wird zur Ablagerung von Lipidmembranen auf Trägern die sogenannte Liposomenfusionstechnik eingesetzt. Dabei wird die Lipidmembran in Form von Lipidvesikel (sogenannte Liposomen) angeboten. In den Liposomen sind vorzugsweise auch bereits die gewünschten funktionellen Moleküle eingebaut. Unter entsprechenden Versuchsbedingungen öffnen sich die Lipidvesikel bei Kontakt mit der Oberfläche des festen Trägers, um schließlich einen zusammenhängenden Lipidfilm auszubilden.

Das SCWP-System kann gegebenenfalls entsprechend Fig. 9 auch dazu verwendet werden, diese Vesikelfusion zu beschleunigen und die Bindung des Lipidfilmes an die SUM zu festigen.

Nutzung von SUM-unterstützten (stabilisierten) funktionellen Lipidmembranen für das High Throughput Screening

Gnindsätzlich bieten sich für die Nutzung zwei Meßaufbauten an:
a) Voltage Clamp- oder Patch Clamp Methoden
b) Black Lipid Membrane Methoden

Die Verwendung von SUMs soll in beiden Fällen die Lebenszeit und damit die Verwendbarkeit von funktionellen Lipidmembranen verlängern.

### Herstellung von SUM-Röhrchen

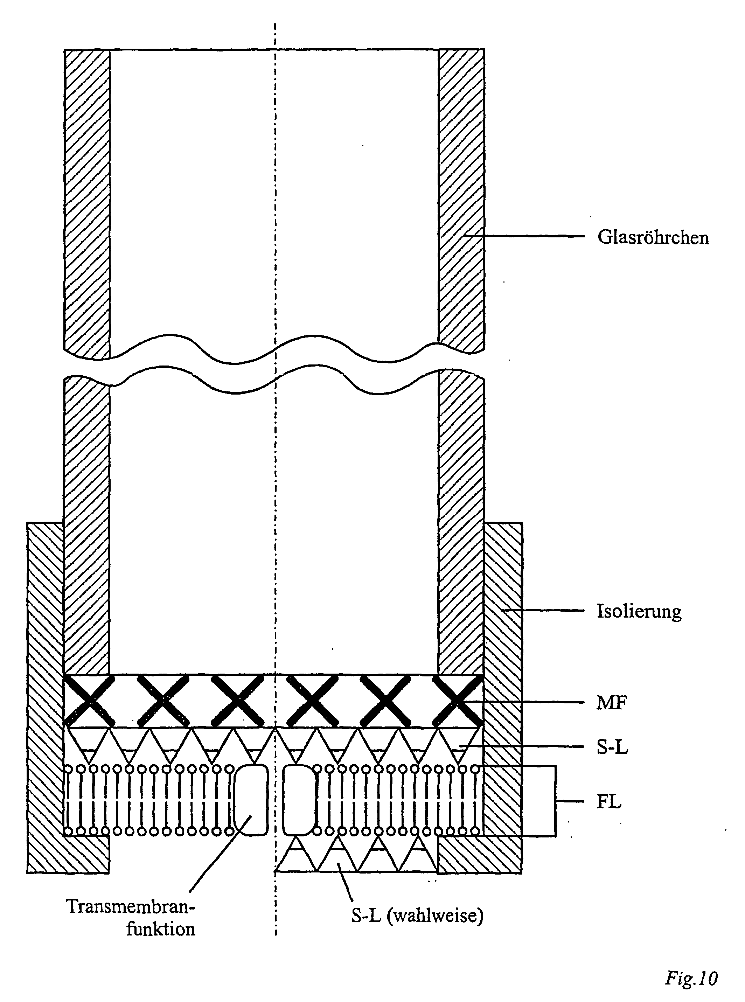

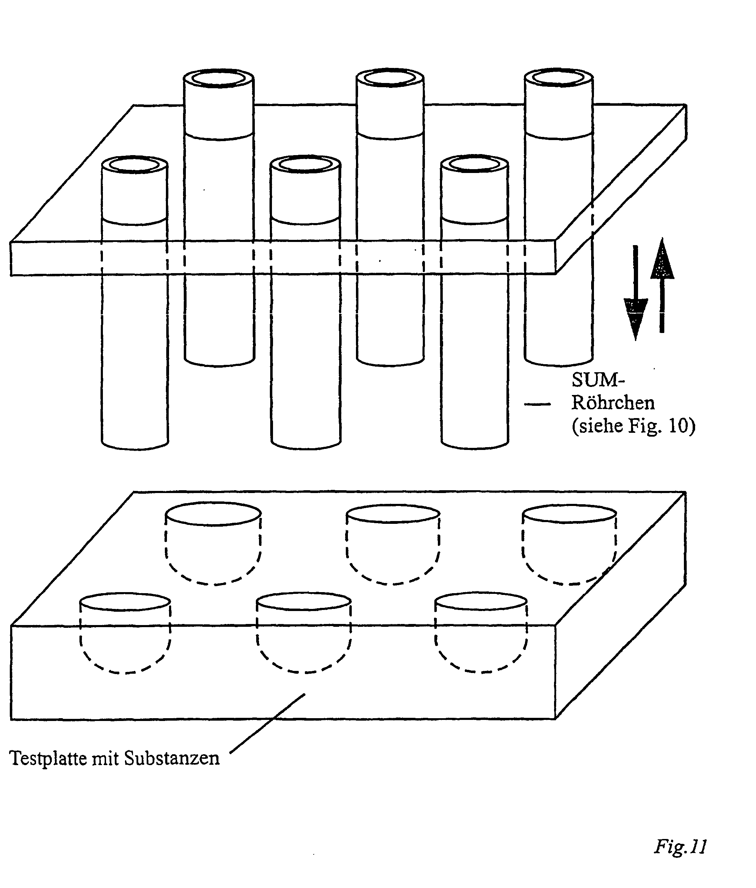

Entsprechend Fig. 10 soll die SUM am Ende eines Glas- oder Kunststofiröhrchens so montiert werden, daß eine SUM-überspannte Apertur entsteht. Dieser Teil würde im Prinzip der Öffnung einer Patch Clamp- oder Voltage Clamp Pipette entsprechen; mit dem Unterschied, daß die (funktionelle) Lipidmembran nicht die Pipettenöffnung freitragend überspannt, sondern durch die SUM unterstützt wird. Das Aufbringen der Lipidmembran und ihre Funktionalisierung könnte nach den verschiedenen bereits oben beschriebenen Methoden erfolgen.

Ein weiterer Vorteil bei der Verwendung von SUM-Röhrchen wird darin gesehen, daß nach der Aufbringung der Lipidmembran eine weitere S-Schicht durch Ablagerung oder Rekristallisation (siehe Fig. 7 und Fig. 8) aufgebracht werden könnte. Diese zusätzliche S-Schicht würde sowohl eine (weitere) Stabilisierungs- als auch Schutzfunktion erfüllen. In diesem Zusammenhang wird an die scharfe molekulare Trenngrenze der S-Schichten erinnert.

Nach der Aufbringung der Lipidmembran auf die SUM bzw. vor oder nach einer eventuellen Rekristallisation einer weiteren S-Schicht kann eine inter- und/oder intramolekulare chemische Vernetzung der S-Schicht (Glykoprotein) Matrix und des Lipidfilmes erfolgen. Dazu müßten entsprechende Crosslinker in die wässrige Phase eingebracht werden.

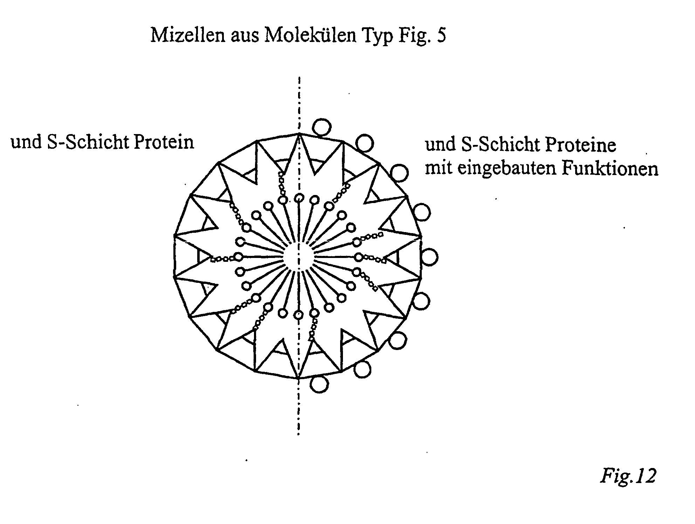

Die SUM-Röhrchen können entweder einzeln oder in Gruppenanordnungen (siehe Fig. 11) verwendet werden. Eine Anordnung in Gruppen ermöglicht die Aufbringung der Lipidmembranen in einem Arbeitsschritt und in der Folge Parallelmessungen in einer beliebigen Vielzahl von Gefäßen, in denen sich unterschiedliche Substanzen befinden, deren Wirkung auf die funktionalisierten Membranen geprüft werden soll. Bei der Verwendung von SUM-Röhrchen könnten auch direkt in die mit Pufferlösungen oder H₂O gefüllten Glasröhrchen Wirksubstanzen zugesetzt werden. Weiters könnten, so erforderlich, die Milieubedingungen (z.B Ionenstärke, Ionenzusammensetzung, pH-Wert, Temperatur) in den Röhrchen und getrennt davon in den Gefäßen, in die sie eintauchen, verändert werden, ohne durch eventuell auftretende Druckschwankungen die Integrität des funktionellen Lipidfilmes zu beeinträchtigen. Gerade diese eingangs bereits erwähnte erhöhte Stabilität der SUMassoziierten Lipidmembranen gegenüber Druckveränderungen ist beim erfindungsgemäßen Einsatz besonders nennenswert. Die oben angeführten Vorteile lassen sich auch direkt auf die Meßanordnungen der Black Lipid Membranen übertragen.

Bei Konjugaten aus einem SCWP-(Kohlenhydrat-)Anteil und einem funktionellen Molekülteil kann der Kohlenhydratanteil aus einem intakten SCWP, aus durch chemischen Abbau aus SCWP hergestellten Partialstrukturen (Oligosaccharidfragmente) oder aus synthetisch hergestellten Partialstrukturen der Scwp (Oligosaccharid-Derivaten) bestehen.

Der funktionelle Molekülteil kann dabei als Funktion eine Bindung an Lipidphasen (z.B.Liposomen) oder Gold, eine feste Verankerung an einer Festphase, z.B. S.-Schicht), oder eine Polymerisierbarkeit (das gebildete Konjugat wirkt als Ko-Monomer), weiters eine pharmazeutische Wirkung, ferner eine kombinierte Funktion, wie Bindung an Lipidphasen und immunstimulierende Wirkung (Lipid-A-Derivate, Lipopeptide vom Typ FK-565) oder schließlich eine Detektierbarkeit (z.B. Fluoreszenz) haben.

Chemisch kann die Verknüpfung von Kohlenhydratanteil und funktionellem Anteil auf verschiedene Weise erfolgen, nämlich durch
reduktive Aminierung (Bildung von Schiff'schen Basen mit nachfolgender Reduktion zu sekundären Aminen,
Thiohanstoffbildung oder
Amidbildung.

Die reduktive Aminierung kann an der Carbonylkomponente am Kohlenhydratteil durch Abspaltung des reduzierenden Phosphorsäurerestes vom SCWP unter Bildung von reduzierendem SCWP, durch partielle De-N-Acetylierung und Desaminierung mit salpetriger Säure unter Bildung von Anhydromannose (Anhydroglucose?)-Endgruppen, durch β-Eliminerung von Uronsäure-hältigem SCWP, oder durch Spaltung mit Perjodat (eventuell partielle Spaltung) erfolgen. An der Aminokomponente am funktionellen Teil kann die reduktive Aminierung durch Phosphatidylethanolamin, durch Ethylendiamin, Propoylendiamin bzw. Einführung einer Aminogruppe am reduzierenden Ende des SCWP, durch Allylamin, Aminomethacrylsäure und deren Derivate, andere Aminoverbindungen mit ungesättigten Gruppen (Aldrich-Katalog) oder S-layers (ε-Lysin-NH₂-Gruppen) vorgenommen werden. Aminogruppen am Kohlenhydratteil können durch Reaktion einer Carbonyl-Komponente mit Ethylendiamin oder anderen Diaminoverbindungen entstehen. Die Carbonylkomponente an funktionellen Teil kann als Smith-Produkt von S-Schicht oder von Polysacchariden erhalten werden.

Die Thioharristoffbildung kann mittels FITC (Fluoresceinisothiocyanat) oder durch Umwandlung von primären Aminen in Isothiocyanate durch Reaktion mit Thiophosgen erfolgen.

Die Amidbildung kann an dem Carbonsäureanteil am Kohlenhydrat (SCWP) mittels Bromoxidation Lactal-Lacton, an der Aminokomponente analog der reduktiven Aminierung, an der Aminokomponente am Kohlenhydrat (SCWP) durch Bildung durch Reaktion einer Carbonylkomponente mit Ethylendiamin oder einer anderen Diaminoverbindung, und am Carbonsäureteil am fuktionellen Molekül als Aktivester von langkettigen Fettsäuren und verzweigten Fettsäuren, als Aktivester von Spargelsäure, oder Liponsäure, sowie als Aktivester von Methacrylsäure(derivaten) und anderen ungesättigten Carbonsäuren erfolgen.

### BEISPIELE:

### Gruppe 1: Modifikation der sekundären Zellwandpolymere

Die sekundären Zellwandpolymere (SCWP) wurden aus peptidoglykanhältigen Sacculi von verschiedenen Bacillaceae unter Verwendung von 48 % iger Flußsäure (HF) nach der Vorschrift von Ries et al. (1997) extrahiert, mittels Gelchromatographie gereinigt, die Eluate gegen A. purif. dialysiert, die Innendialysate bei -20°C eingefroren und lyophilisiert. Die verwendeten Organismen, deren S-Schicht-Proteine und SCWP haben folgende Bezeichnung: *Bacillus stearothermophilus* PV72/p6 (Sära et al., 1996), SbsA (Kuen et al., 1994), Typ A SCWP (Egelseer et al., 1998); *Bacillus stearothermophilus* ATCC 12980 (Egelseer et al., 1996), SbsC (Jarosch et al., 2000), Typ A SCWP (Egelseer et al., 1998); *B*. *stearothermophilus* PV72/p2 (Sára et al., 1996), SbsB (Kuen et al., 1997), Typ B SCWP (Ries et al., 1997; Sára et al., 1998); *B. sphaericus* CCM 2177 (Sára et al., 1989), SbpA, Typ C SCWP (Ilk et al., 1999); *Thermoanaerobacter thermohydrosulfuricus* L111-69 (Sára et al., 1988); Stta, Typ D SCWP. Eine genaue Auflistung der Eigenschaften der einzelnen S-Schicht-Proteine und der entsprechenden SCWP findet sich in der folgenden Tabelle 1.

**TABELLE 1**

| Organismus | 1. S-Schicht-Protein 2. Gittertyp 3. Gesamtlänge 4. Signalpeptid | GenBank Accession Number | SCWP | Hauptkomponenten des SCWP / maximales MG | Interaktion des SCWP mit dem |
|---|---|---|---|---|---|
| *B*. *stearothermophilus* PV72/p6 | SbsA hexagonal 1 228 30 | X 71092 | Typ A | Glukose : N-Acetylglukosamin : 2,3-Dideoxy-2,3-Diacetamido-mannuronsäure = 1 : 1 : 2; Struktur aufgeklärt (MGₘₐₓ 50,000) | N-Terminus (AS 31-257) keine SLH-Motife |
| *B. stearothermophilus* ATCC 12980 | SbsC schräg 1 099 30 | AF055578 | Typ A | ident wie oben | N-Terminus (AS 31-257) keine SLH-Motife |
| *B. stearothermophilus* Mut 1 | SbsD schräg 940 30 | AF228338 | Typ A | ident wie oben | N-Terminus (AS 31-257) keine SLH-Motife |
| *B*. *stearothermophilus* PV72/p2 | SbsB schräg 920 31 | X 98095 | Typ B | N-Acetylglukosamin : N-Acetylmannos-amin = 2: 1; durch Pyruvatketale negativ geladen, Glycerin und Uronsäuren vorhanden; sehr komplex aufgebaut; Struktur noch nicht vollständig aufgeklärt (MGₘₐₓ ∼ 24,000) | N-Terminus (*3 SLH- Motife; AS 33-204)* |
| *B*. *sphaericus* CCM 2177 | SbpA quadratisch 1 322 30 | AF211170 | Typ C | N-Acetylgtukosamin : N-Acetylmannosamin = 2 : 1, jeder 2. N-Acetylmannosamin rest trägt ein Pyruvatketal; Struktur aufgeklärt (MGₘₐₓ ∼ 10,000) | N-Terminus (3 SLH-Motife AS 33-202) |
| *Th*. *thermohydrosulfuricus* L111-69 | SttA hexagonal nicht bekannt | keine | Typ D | N-Acetylglukosamin : N-Acetylmannosamin : Mannose = 1 : 0.5 : 1; durch Pyruvatketale negativ geladen; Struktur noch nicht aufgeklärt (MGₘₐₓ ∼ 25,000) | N-Terminus |

### Beispiel 1: Konvertierung der latenten Aldehydgruppe (reduzierendes Ende) der Polymerketten in freie Aminogruppen

Zur Modifikation des reduzierenden Endes (latente Aldehydgruppe) der SCWP vom Typ A, B, C und D wurde folgendermaßen vorgegangen: 10 mg des lyophilisierten Materiales wurden in 2 ml gesättigter Carbazolhydrazidlösung (pH 6.2) gelöst. Anschließend wurden 200 µl Natriumcyanoborhydrid (NaBH₃CN; 20 mg / ml A. purif.) zugefügt, 15 Minuten bei 100°C, dann 16 h bei 90°C inkubiert. Zur Entfernung der überschüssigen Reagentien wurde 24 h gegen A. purif. dialyisert, die Proben bei -20°C eingefroren und lyophilisiert. Zur Bestimmung der freien Aminogruppen wurden 1 mg des jeweiligen SCWP in 2 ml 50 mM Natriumhydrogencarbonat-Puffer (pH 7.8) gelöst, und 100 µl einer Lösung von Butyloxycarbonyl-L-Leucin-N-Hydroxysuccinimidester (BOC-Leuse; 3.5 mg / ml 100 % igem Ethanol) zugefügt. Der Reaktionsansatz wurde 18 h bei 20°C gerührt, dann 6 h gegen eine Mischung aus Ethanol - A. purif. (30 : 70 / v : v) und anschließend gegen A. purif. dialysiert. Das Innendialysat wurde in der Folge lyophilisiert und 0.5 mg des lyophilisierten Materiales mit 6 N HCl 6 h bei 110°C für die Aminosäure- und Aminozuckemalyse (Aminosäureanalysator Biotronik; Maintal, D) hydrolysiert. Aus dem Verhältnis von Glukosamin zu Leucin konnte der Modifikationsgrad der Polymerketten bestimmt werden, der für alle SCWPs > 95 % war.

### Beispiel 2: Konvertierung der in die SCWP eingeführten Aminogruppen in Thiolgruppen

Zur Konvertierung der in die SCWP eingeführten Aminogruppen wurden 5 mg der nach Beispiel 1 modifizierten SCWP in 5 ml 0.25 M Triethanolamin-HCl-Puffer (pH 8.5) gelöst. Nach Zugabe von 4 mg 2-Iminothiolan (4-Mercaptobutyrimidat) wurde die Lösung 2 h bei 37°C unter Stickstoff-Atmosphäre inkubiert, und anschließend zur Entfernung der Reagentien 18 h gegen entgastes A. purif. bei 4°C dialysiert. Das Innendialysat wurde in der Folge eingefroren und lyophilisert. Die Anzahl der eingeführten Thiolgruppen wurde nach der Methode von Ellman (1959) bestimmt. Es zeigte sich, daß die Aminogruppen quantitativ in Thiolgruppen konvertiert werden konnten.

### Beispiel 3: Modifikation der eingeführten Aminogruppen mit Biotin

Zur Modifikation der freien Aminogruppen (siehe Beispiel 1) wurde 5 mg lyophilisiertes SCWP vom Typ A, B, C oder D in 20 mM Kalium-Phosphat-Puffer (pH 7.8) gelöst und 0.5 mg Sulfo-NHS-Biotin (Sigma) zugegeben. Die Modifikation erfolgte nach der von der Firma Pierce empfohlenen Vorschrift. Um überschüssige Reagentien zu entfernen, wurde 18 h bei 4°C gegen A. purif. dialysiert, das Innendialysat bei -20°C eingefroren und lyophilisiert.

### Beispiel 4: Aktivierung der eingeführten freien Aminogruppen

Zur Aktivierung der eingeführten Aminogruppen wurden 5 mg der Lyophilisate aus Beispiel 1 in 500 µl einer Lösung aus p-Phenylendiamin (10 mg / ml A. purif.) aufgelöst. In der Folge wurden 100 µl NaBH₃CN-Lösung (20 mg / ml A. purif.) zudosiert, 15 min bei 100°C, dann 16 h bei 90°C inkubiert. Die Reagentien wurden anschließend durch Dialyse (18 h, 4°C) gegen A. purif. oder wahlweise durch Reinigung über eine Superdex S75-Säule (Pharmacia, Uppsala, Schweden) mit 0.1 M TRIS-HCl-Puffer (pH 7.8) als Laufmittel entfernt. Die Diazotierung der in die SCWPs eingeführten Aminogruppen erfolgte mit NaNO₂ / HCl nach der von Manjon (1985) bschriebenen Methode. Zur Diazotierung wurden 5 mg mit p-Phenylendiamin modifiziertes SCWP in 35 µl einer vorgekühlten Mischung aus 2 Teilen einer NaNO₂-Lösung (4 % in A. purif.) und 5 Teilen 2 N HCl 1 h bei 4°C gerührt. Überschüssiges Reagens wurde durch Dialyse gegen A. purif. (4 h, 4°C) entfernt, und die Lösung mit dem diazotierten SCWP sofort mit den Substanzen, die freie Aminogruppen enthielten, in Kontakt gebracht.

### Beispiel 5: Nachweis der biologischen Aktivität der HF-extrahierten, nativen und chemisch modifizierten SCWP

Zum Nachweis der spezifischen Bindung zwischen den in der Einführung genannten S-Schicht-Proteinen und den verschiedenen Typen von SCWPs wurde die "Surface Plasmon Resonance Technique" (SPR) angewendet (Gerät: Biacore 2000, Biacore, Uppsala, Schweden). Die S-Schicht-Proteine wurden in wasserlöslicher Form als nicht assemblierte Monomere und / oder Oligomere in einer Konzentration von 100 µg / ml A. purif. auf voraktivierten Carboxy-Dextran-Gold-Chips immobilisiert. Die Resonanz-Units ergaben, daß die Oberfläche der Chips zu etwa 70 % mit S-Schicht-Protein bedeckt war, wobei aufgrund der angewendeten Methode eine Immobilisierung der Subeinheiten in statistischer Orientierung erfolgte. Beispielhaft werden die Untersuchungen mit dem S-Schicht-Protein SbsB von *B. stearothermophilus* PV72/p2, dem rekombinant in *Escherichia coli* hergestellten S-Schicht-Protein (r SbsB₃₂₋₉₂₀; entspricht dem reifen SbsB des Wildtypstammes), und einer N-terminal verkürzten Form (r SbsB₂₀₈₋₉₂₀; es fehlen die drei SLH-Motife) dargestellt. Aufgrund der Adsorptionskurven zeigte sich, daß auf Chips immobilisiertes
SbsB und r SbsB das Typ B SCWP (Konzentrationsbereich 1 - 100 µg / ml 50 mM TRIS-HCl-Puffer, pH 7.2) spezifisch erkennen;
r SbsB₂₅₈₋₉₂₀ (= Δ 3 SLH-Motife) keine Affinität zum Typ B SCWP besitzt;
SbsB und r SbsB₃₂₋₉₂₀ zu Typ B SCWP, das nach Beispiel 1 bis 4 modifiziert worden war, eine vergleichbare Affinität wie zu nativen Typ B SCWP hatten;
SbsB und r SbsB₃₂₋₉₂₀ HF-extrahiertes, mit Lysozym verdautes Peptidoglykan nicht binden.

Vergleichende Versuche wurden auch mit den S-Schicht-Proteinen SbsA, SbsC und r SbsC₃₁₋₁₀₉₉ und dem Typ A SCWP, sowie mit SbpA, r SbpA und dem Typ C SCWP und SttA und dem Typ D SCWP druchgeführt. In allen Fällen konnte der eindeutige Nachweis der spezifischen Bindung zwischen dem jeweiligen S-Schicht-Protein und dem dazugehörigen SCWP erbracht werden.

### Beispiele der Gruppe 2: Bindung der modifizierten SCWPs an Moleküle eines festen Trägers zur orientierten monomolekularen Bindung von S-Schicht-Proteinen

### Beispiel 6: Verwendung von Mikrofiltrationsmembranen (MF) als festen Träger

Als Träger wurde eine MF (Pall Carboxydyne) mit einer durchschnittlichen Porengröße von 0.4 µm und einem Durchmesser von 14 mm verwendet. Zur Aktivierung der freien Carboxylgruppen wurde die MF in eine Lösung aus EDC (10 mg / ml A. puif., pH 4.7) 1 h bei 20°C gelegt, und anschließend dreimal mit eiskaltem A. purif. gewaschen. Zur kovalenten Bindung des Typ C SCWP wurden 5 mg des unter Beispiel 1 beschriebenen, modifizierten Materiales in 5 ml A. purif. gelöst, wobei der pH Wert der Lösung mit 0.01 N NaOH auf 9.0 eingestellt wurde. Nach 4 h Inkubation bei 20°C wurde die MF entnommen und mit 50 mM TRIS-HCl-Puffer (pH 7.2) fünfmal gewaschen. Anschließend wurde die MF in eine Lösung von SpbA-Protein (100 µg / ml 10 mM CaCl₂ - Lösung) gelegt und 6 h bei 20°C inkubiert. Zur Entfernung von nicht gebundenem S-Schicht-Protein wurde die MF fünfmal mit 10 ml 50 mM TRIS-HCl-Puffer (pH 7.2) gewaschen und in 1mm² große Stückchen zerschnitten, die mit 100 µl einer SDS-Lösung (Natrium-Dodecylsulfat; 10 % in A. purif.) 10 min bei 100°C extrahiert wurden. In der Folge werden 25 µl des klaren Extraktes mit 75 µl Sample Solution (Laemmli, 1971) gemischt, und 1- 10 µl dieser Mischung auf SDS-Gele (10 % ige Trenngele) aufgetragen. Aufgrund der Stärke der Proteinbanden der Probe (Molekulargewicht von SpbA auf SDS-Gelen 127,000) und dem Vergleich mit entsprechenden SpbA-Standards konnte die Menge an gebundenem S-Schicht-Protein auf 80 - 100 µg /cm² MF geschätzt werden, was der vom Hersteller angegebenen maximalen Bindungskapazität dieser hochporösen MF für Proteine entspricht.

### Beispiel 7: Verwendung eines Siliziumoxid-Wafers (SOW) als festen Träger

SOW mit einer Größe von 3 x 5 mm wurden in 500 µl einer Lösung von Aminosilan (5 % in A. purif., pH 3.5) 30 min bei 37°C inkubiert. Nach fünfinaligem Waschen mit A. purif. und Ethanol, wurden die silanisierten SOW 15 min 110°C getrocknet, schließlich in einer Lösung von m-Maleimidbenzoylsulfosuccinimidester (Sulfo-MBS, Pierce; 150 µg / ml 0.1 M Natrium-Phosphat-Puffer, pH 7.0) 2 h bei 37°C voraktiviert, und mit A. purif. gewaschen. Zur Bindung von modifiziertem Typ B SCWP (wie in Beispiel 2 beschrieben) wurde 1 mg des Lyophilisates / ml 0.1 M Natrium-Phosphat-Puffer (pH 7.0) gelöst, und 500 µl auf den SOW aufgebracht. Nach 2 h Inkubation bei 20°C unter N₂-Atmosphäre wurden die Plättchen mit 0.1 M Natrium-Phosphat-Puffer und A. purif. gewaschen, zur orientierten Bindung entweder SbsB oder r SbsB (100 µg /ml 50 mM TRIS-HCl-Puffer, pH 7.2) angeboten, und der mit Typ B SCWP funktionalisierte SOW 4 h bei 20°C in dieser Lösung inkubiert. Durch Waschen mit TRIS-HCl-Puffer wurde überschüssiges S-Schicht-Protein entfernt, der SOW im AFM untersucht und die Orientierung des schrägen S-Schicht-Gitters bestimmt. Es zeigte sich, daß SbsB und r SbsB mit der Innenseite der Subeinheiten (N-terminale Region, die die 3 SLH-Motife trägt) gebunden hatte. Aus zahlreichen Untersuchungen an SOWs ist bekannt, daß SbsB in Abwesenheit des Typ B SCWP mit der Außenseite bindet (Neubauer et al., 2000). Demnach erfolgte die orientierte Bindung der S-Schicht-Subeinheiten an den SOW über das kovalent an den festen Träger gebundene Typ B SCWP. Anstatt von SbsB kann auch rekombinant hergestelltes SbsB-Streptavidin-Fusionsprotein verwendet werden.

### Beispiel 8: Verwendung eines Siliziumoxid-Wafers (SOW) als festen Träger

Zur Einführung von Thiolgruppen wurde ein 3 x 5 mm großer SOW in einer Lösung aus 3-Mercaptosilan (7 % in 50 % igem Aceton) 30 min bei 37°C gelegt. Nach fünfmaligem Waschen mit 50 % igem Aceton wurden der SOW bei 110°C 15 min lang unter N₂-Atmosphäre getrocknet. Zur Aktivierung der Thiolgruppen wurde 500 µl einer Lösung aus 2-2'-Dipyridyldisulfid (5 mg / ml 10 % igem Ethanol) auf den SOW gebracht. Die Inkubationszeit betrug 2 Stunden bei 20°C. Nach Entnahme des SOW wurde dieser mehrfach mit 10 % igem Ethanol und A. purif. gewaschen und dann in 500 µl einer Lösung aus Typ A SCWP (3 mg / ml A. purif.; modifiziert wie in Beispiel 2 beschrieben) 6 h bei 20°C inkubiert. Zur Entfernung von überschüssigem Typ A SCWP wurde der SOW mehrfach mit A. purif. und 50 mM Natrium-Phosphat-Puffer (pH 7.0) gewaschen. Zur orientierten Bindung wurde der mit Typ A SCWP beladene SOW in eine Lösung aus folgenden S-Schicht-Protein-Formen (100 µg / ml 50 mM TRIS-HCl-Puffer, pH 7.2) für einen Zeitraum von 8 h gelegt: SbsC (Wildtyp-Protein), r SbsC₃₁₋₁₀₉₉, r SbsC₂₅₈₋₁₀₉₉ und r SbsC₃₄₂₋₁₀₉₉. Den letzten beiden Formen fehlte die für das Typ A SCWP verantwortliche N-terminale Bindungsregion (Jarosch et al., 2000). Der SOW wurde nach der Inkubation mit den verschiedenen S-Schicht-Protein-Formen mit TRIS-HCl-Puffer und A. purif. gewaschen und anschließend im AFM untersucht. Aufgrund des schrägen S-Schicht-Gitters, das durch das S-Schicht-Protein SbsC ausgebildet wird, war eine eindeutige Bestimmung der Bindungsseite möglich. Es zeigte sich, daß die Basisvektoren dieselbe Orientierung wie an intakten Zellen oder Zeilwandfragmenten von *B. stearothermophilus* ATCC 12980 hatten. Im Falle der N-terminal verkürzten r SbsC-Formen konnte im AFM keine Gitterstruktur entdeckt werden.

### Beispiel 9: Verwendung von MF als feste Träger

Das Typ A SCWP, das wie in Beispiel 1 beschrieben, modifiziert wurde, wurde an mit Carbodiimid-aktivierte MF gebunden (siehe Beispiel 6). Folgende Formen von SbsC wurden in einer Konzentration von 150 µg / ml 50 mM TRIS-HCl-Puffer (pH 7.2) angeboten, und die Membranscheibchen mit einem Durchmesser von 14 mm 8 h bei 20°C inkubiert: r SbsC₃₁₋₁₀₉₉, r SbsC₂₅₈₋₁₀₉₉, r SbsC_{342-1099,}, r SbsC₃₁₋₈₄₄, r SbsC₃₁₋₈₆₀, r SbsC₃₁₋₈₈₀, r SbsC₃₁₋₉₀₀, r SbsC₃₁₋₉₂₀ und SbsC. Nach der Inkubation wurden die MF mit 50 mM TRIS-HCl-Puffer (pH 7.2) gewaschen, in 1 mm² große Teilchen zerschnitten, mit SDS-Lösung extrahiert und mittels SDS-PAGE untersucht (siehe Beispiel 6). Aufgrund der entsprechenden Standardreihen konnte die gebundene Menge an jenen Formen, die den vollständigen N-Terminus trugen, zwischen 80 - 100 µg / cm² MF abgeschätzt werden. Hingegen fand im Falle von r SbsC₂₅₈₋₁₀₉₉ und r SbsC₃₄₂₋₁₀₉₉ keine Bindung an die mit Typ A SCWP funktionalisierte MF statt. Der Einsatz der unterschiedlich verkürzten Formen bestätigte die Ergebnisse der Affinitätsstudien (Egelseer et al., 1998; Jarosch et al., 2000) und der SPR-Messungen, daß ausschließlich der N-terminus für die Interkation mit dem SCWP verantwortlich ist.

### Beispiele der Gruppe 3: Synthese von Glyko-Lipiden (GL) aus SCWPs

### Beispiel 10: GLs aus allen Typen von SCWPs

Die lyophilisierten nativen SCWPs wurden in A, purif. gelöst (10 mg / ml A. purif.) und mit einer Lösung aus Dipalmitoylphosphatidylethanolamin (DPPE ; 20 mg / 400 µl Chloroform : Methanol = 1 : 1) vermischt. Nach Inkubation in verschraubten Reaktionsröhrchen bei 60°C über einen Zeitraum von 4 h wurde der Lösung 4 mg NaBH₃CN hinzugefügt, und die Inkubation 16 h bei 60°C fortgesetzt. Die gebildeten GLs wurden mittels Gelchromatographie (Superdex S75) gereinigt. Das Säuleneluat wurde auf Glukosamin und Phosphat überprüft; jene Fraktionen, die beide Komponenten enthielten, wurden vereinigt und lyophilisiert.

### Beispiel 11: GLs aus allen Typen von SCWPs

Anstatt der SCWP mit reduzierendem Ende wurden die nach Beispiel 2 modifizierten SCWPs mit einer freien Thiolgruppe eingesetzt. Als Lipidkomponente wurde DPPE verwendet. Zur Aktivierung der SCWPs wurden jeweils 5 mg in 1 ml 50 mM Natrium-Phosphat-Puffer (pH 7.0) gelöst, und 1 mg Sulfo-MBS hinzugefügt. Nach Inkubation für 2 h bei 37°C wurde die Reaktionsmischung mit einer Lösung von DPPE (3 mg in 500 µl Chloroform : Methanol = 1: 1 = v : v) vermischt, und der Ansatz 16 h bei 20°C unter Rühren inkubiert. Die Reinigung der GLs erfolgte, wie in Beispiel 10 beschrieben, gelchromatographisch mit Hilfe einer Superdex S75 - Säule.

### Beispiele der Gruppe 4: Verwendung der GLs zur Herstellung von Liposomen und zur orientierten Bindung der S-Schicht-Proteine

### Beispiel 12: Nachweis des Einbaus von GLs in Liposomen

Lipsomen, die DPPC, Cholesterin und GL nach Beispiel 10 oder 11 aus Typ C SCWP in einem molaren Verhältnis von 10 : 5 : 4 enthielten, wurden nach der von Mader et al. (1999) beschriebenen Methode hergestellt und mittels Gelchromatographie von den Ausgangskomponenten gereinigt. Zum Nachweis, ob die hydrophilen Ketten des Typ B SCWP an der Oberfläche der Liposomen exponiert waren, wurde eine Perjodatoxidation, bei der nur vicinale Hydroxylgruppen angegriffen werden, durchgeführt. Die Liposomen, die für die Reaktion eingesetzt wurden, entsprachen einer DPPC-Konzentration von 2 µmol / ml 0.2 M Natrium-Acetat-Puffer (pH 4.5). Dieser Suspension wurde soviel Natriumperjodat zugegeben, daß eine Endkonzentration von 20 mM erreicht wurde. Die Reaktionsmischung wurde 1 Stunde im Dunkeln bei 20°C unter Rühren inkubiert. Nach Zentrifugieren und zweimaligem Waschen der Liposomen mit 0.2 M Natriumhydrogencarbonat-Lösung (pH 8.2) wurden diese in 2 ml einer Lösung aus Ferritin (1 mg / ml 0.2 M Natrimhydrogencarbonat, pH 8.2) 4 h bei 20°C inkubiert. Die Liposomen wurden anschließend mehrmals mit Natriumhydrogencarbonat-Lösung gewaschen. Für Untersuchungen im Transmissions-elektronenmikroskop wurden die Proben sowohl negativkontrastiert, wie auch für Ultradünnschnitte eingebettet. Im negativkontrastierten Präparat zeigte sich, daß die Liposomenoberfläche vollständig mit Ferritin bedeckt war; im Ultradünnschnitt konnte eine Monoschicht von Ferritin an der Liposomenoberfläche nachgewiesen werden. Nach Perjodatoxidation war das Typ B SCWP nicht mehr biologisch aktiv; die freien Aldehydgruppen konnten jedoch zur kovalenten Bindung von Fremdmolekülen mit exponierten Aminogruppen herangezogen werden.

### Beispiel 13: Orientierte Rekristallisation des S-Schicht-Proteins SbsB

Die unter Beispiel 12 beschriebenen Liposomen, die das GL aus DPPE und Typ B SCWP enthielten (siehe Beispiel 10), wurden zur orientierten Rekristallisation des S-Schicht-Proteins SbsB eingesetzt. Auf Liposomen, die DPPE und Cholesterol enthielten, bindet SbsB mit der Außenseite, sodaß dert N-Terminus mit der Bindungsregion für das Typ B SCWP in das externe Milieu gerichtet ist (Mader et al., 1999). Im Falle der Liposomen, die das GL aus DPPE und Typ B SCWP enthielten, wurden 2 ml einer Suspension mit einem DPPE-Gehalt von insgesamt 4 µmol mit 2 ml einer SbsB-Lösung (1 mg / ml 50 mM TRIS-HCl-Puffer, pH 7.2) 6 h bei 20°C inkubiert, die Suspension zur Entfernung von überschüssigem S-Schicht-Protein zentrifugiert, die Liposomen mit 50 mM TRIS-HCl-Puffer (pH 7.2) gewaschen, und die Präparate nach einer Gefriertrockung bei -80°C und Schrägbeschattung mit Pt/C im Transmissionselektronenmikroskop untersucht. Es zeigte sich, daß im Falle der GL-hältigen Liposomen die glatte Außenseite des schrägen S-Schicht-Gitters exponiert war, während bei den DPPE-Cholesterol-Liposomen die rauhe Innenseite zu erkennen war.

### Beispiel 14: Modifikation von DPPC/Cholesterol/HDA-hältigen Liposomen mit SCWP

Liposomen wurden wie von Mader et al. (1999) beschrieben, hergestellt. Das unter Beispiel 2 beschriebene, mit 2-Iminothiolan modifizierte Typ C SCWP wurde zum Funktionalisieren der Liposomenoberfläche verwendet. Aus diesem Grunde wurden die Liposomen, die einer DPPC-Konzentration von 5 µmol entprachen, in 3 ml 0.2 M Natrium-Phosphat-Puffer (pH 7.0) suspendiert, und 2 mg Sulfo-MBS zur Aktivierung der freien Aminogruppen von HDA dazugegeben. Nach einer zweistündigen Inkubation bei 20°C wurde zentrifugiert, die Liposomen in 2 ml einer Lösung aus Typ C SCWP (1.5 mg / ml Natrium-Phosphat-Puffer, pH 7.0; modifiziert nach Beispiel 2) suspendiert, und 18 h bei 20°C inkubiert. Der Nachweis der Immobilisierung des SCWP erfolgte, wie in Beispiel 12 beschrieben, über Perjodatoxidation und Immobilisierung von Ferritin. Die Rekristallisation des S-Schicht-Proteins SbpA wurde analog der Vorgangsweise in Beispiel 13 durchgeführt. Der Nachweis der orientierten Bindung des SbpA-Proteins erfolgte elektronenmikroskopisch an gefriergetrockneten und mit Pt/C-bedampften Präparaten.

### Beispiel 15: Verwendung des r SbsB-Streptavidin-Fusionsproteines

Ein C-terminales rekombinant in *E*. *coli* hergestelltes SbsB-Streptavidin-Fusionsprotein (4 mg Lyophilisat) wurde in 2 ml 6 M Guanidinhydrochlorid in 50 mM TRIS-HCl-Puffer (pH 7.2) gelöst, 0.8 mg Streptavidin zugegeben, und die Lösung 1 h bei 20°C gegen A. purif. dialysiert. Nach Entnahme des Innedialysates wurde bei 40,000 x g zentrifugiert, und der klare Überstand, der lösliches r SbsB-Strept(4) enthielt, auf eine Proteinkonzentration von 120 µg / ml 0.2 TRIS-HCl-Puffer (pH 7.2) eingestellt. 1 ml der Proteinlösung wurde in der Folge mit 1 ml der Liposomensuspension, die nach Beispiel 13 hergestellt worden war, vermischt, und die Proben 2 h bei 20°C inkubiert. Nach Zentrifugieren wurden die Liposomen zweimal mit 0.2 M TRIS-HCl-Puffer (pH 7.2) gewaschen, schließlich mit biotiniliertem human IgG inkubiert und die Bindung nach der von Mader et al. (2000) entwickelten Vorschrift überprüft. Eine Bindung von biotiniliertem human IgG war nur aufgrund der an der S-Schicht-Außenseite exponierten Streptavidin-Reste möglich. Die gewünschte Orientierung (Bindung über den an der S-Schicht-Innenseite liegenden N-Terminus) wurde nur für jene Liposomen beobachtet, bei denen das Typ B SCWP kovalent an HDA gebunden worden war.

### Beispiele der Gruppe 5: Aufbau von Lipiddoppelschichten

### Beispiel 16: Lipid-Bilayer mit Kanalprotein mit einem Silizium-Wafer als feste Oberfläche

Auf einem SOW (3 x 5 mm) wurden durch Silanisieren mit Mercaptosilan (siehe Beispiel 8) Thiolgruppen eingeführt, an die mittels Sulfo-MBS als heterobifunktionellen Crosslinker nach Beispiel 1 modifiziertes Typ A SCWP (1 mg / ml 50 mM Kalium-Phosphat-Puffer, pH 7.0) gebunden wurde. Nach gründlichem Waschen des SOW mit Phosphatpuffer und A. purif. wurden 500 µl einer Lösung aus SbsA-Protein (100 µg / ml 50 mM TRIS-HCl-Puffer, pH 7.2) aufgebracht, 6 h bei 20°C inkubiert, mit Puffer gewaschen und das als Monoschicht vorliegende SbsA-Protein mit Glutaraldehyd (0.5 % in 50 mM Kalium-Phosphat-Puffer, pH 7.2) 20 min bei 20°C vemetzt. In der Folge wurde ein Bilayer aus DPPE nach der von Schuster et al. (1998) beschriebenen Methode aufgebracht, in dem α-Haemolysin oder Valinomycin inseriert worden waren (Schuster et al., 1998 a,b).

### Beispiel 17: Verwendung einer S-Schicht-Ultrafiltrationsmembrane (SUM) als festen Träger

Ein SUM-Scheibchen (Durchmesser 25 mm) wurde in eine Ultrafiltrationszelle eingelegt, sodaß nur die S-Schicht-Außenseite exponiert war. Die freien Carboxylgruppen des S-Schicht-Proteins wurden durch Zugabe einer EDC-Lösung (6 mg / ml A. purif.; pH 4.7) 80 min bei 20°C aktiviert, die Membranoberfläche dreimal mit eiskaltem A. purif. gewaschen, und anschließend 2 ml einer Lösung des nach Beispiel 1 modifizierten Typ A SCWP (1 mg / ml; pH 9.0) zur kovalenten Bindung an der S-Schicht-Außenseite aufgebracht. Nach 5 h Inkubation bei 20°C wurde die SUM dreimal mit 50 mM TRIS-HCl-Puffer (pH 7.2) und dreimal mit A. purif. gewaschen, und schließlich mit einer Lösung von SbsA-Protein (100 µg / ml 50 mM TRIS-HCl-Puffer, pH 7.2) 5 h bei 20°C inkubiert. Nach Waschen des Membranscheibchens mit Puffer wurde ein Teil zerkleinert, und zur Untersuchung der gebundenen SbsA-Menge mittels SDS-PAGE mit SDS-Lösung extrahiert. Eine im Parallelverfahren hergestellte SbsA-SCWP-SUM wurde zur Stabilisierung des S-Schicht-Proteins mit Glutaraldehyd (wie in Beispiel 16 beschrieben) vemetzt, und zur Beschichtung mit einem DPPE-Film und integralen Membranproteinen (z. B. α-Hameolysin) verwendet.

### Beispiel 18: Kovalente Bindung eines GL an die SUM

Verwendung einer SUM und Beschichtung mit dem im Beispiel 10 oder 11 beschriebenen GL aus Typ C SCWP und DPPE. In der Folge wurde die mit Typ C SCWP funktionalisierte SUM mit einer Lösung aus SbpA-Protein (50 µg / ml 10 mM CaCl₂) 6 h bei 20°C inkubiert, und die SUM-Oberfläche mit 10 mM CaCl₂-Lösung gründlich gewaschen. Zur Überprüfung der orientierten monomolekularen Bindung des SbpA-Proteins wurde die SUM für Untersuchungen im Transmissionselektronenmikroskop sowohl einer Gefriertrocknung unterzogen, wie auch für Ultradünnschnitte verwendet. Es zeigte sich, daß eine geschlossene monomolekulare Schicht mit quadratischem Gitter vorhanden war, wobei die glatte Außenseite des S-Schicht-Gitters exponiert war.

### Beispiele der Gruppe 6: Herstellung von SCWP-haltigen Kopolymeren

### Beispiel 19: Herstellung eines Kopolymers unter Verwendung von Acrylamid

Eine Lösung, die pro ml A. purif. 20 mg nach Beispiel 1 modifiziertes Typ C SCWP, 6.8 mg Acrylamid und 3 µl N,N,N',N'-Tetramethylethylendiamin enthält, wird 30 min am Wasserstrahlvakuum entgast. Nach Zugabe von Ammonperoxodisulfat (1 mg) wird 18 h bei 4°C reagieren gelassen. Anschließend wird das Produkt über Sephadex G-50 getrennt (2,6 × 100 cm, 0,01 M Natriumhydrogencarbonat), über Biogel P2 entsalzt und lyophilisiert. Der Gehalt an SCWP wird über Aminozucker-Analyse ermittelt.

### Beispiel 20: Herstellung eines Kopolymers unter Verwendung von Polyvinylalkohol

Es wird eine Lösung von Polyvinylalkohol (PVA; 3 % in A. purif.) hergestellt und zu 1 ml dieser Lösung werden 20 mg natives Typ A SCWP dazugefügt. Anschließend werden 100 µl auf einem gereinigten Objektträger ausgegossen, und zur Stabilisierung des Filmes dieser 30 Minuten bei 37°C getrocknet. Alternativ kann der Film durch Inkubation in einer Lösung aus Glutaraldehyd (0.5 % in 0.1 M Natrium-Phosphat-Puffer, pH 7.2) über einen Zeitraum von 10 Minuten chemisch fixiert werden. Der mit A. purif. gewaschene PVA-SCWP-Film wird als Matrix zur orientierten Rekristallisation des S-Schicht-Proteins SbsC verwendet (siehe Beispiel 8 und 9)

## Patentansprüche

1. Verwendung von extrahiertem, gereinigtem sekundärem Zellwandpolymer prokaryontischer Mikroorganismen zur orientierten monomolekularen Bindung von (funktionellen) Molekülschichten und/oder deren Anlagerung an Molekülen eines Trägers.

2. Verwendung nach Anspruch 1, **dadurch gekennzeichnet, daß** die Bindung der sekundären Zellwandpolymere über entsprechende Domänen der funktionellen Molekülschichten und/oder des Trägers erfolgt.

3. Verwendung nach Anspruch 1 oder 2, **dadurch gekennzeichnet, daß** die Bindung der sekundären Zellwandpolymere an den Molekülschichten und/oder den Molekülen des Trägers über lektinartige Bindungen erfolgt.

4. Verwendung nach einem der Ansprüche 1 bis 3, **dadurch gekennzeichnet,daß** die sekundären Zellwandpolymere die Bindeglieder zwischen polymeren Trägerstrukturen und funktionellen Molekülen bzw. Molekülgruppen sind.

5. Verwendung nach 4, **dadurch gekennzeichnet, daß** die polymeren Trägerstrukturen Mikrofiltrationsmembranen sind.

6. Verwendung nach Anspruch 4 oder 5, **dadurch gekennzeichnet, daß** die Molekülschichten zweidimensionale kristalline Proteinschichten sind.

7. Verwendung nach Anspruch 6, **dadurch gekennzeichnet, daß** die zweidimensionalen kristallinen Proteinschichten ihrerseits Träger einer funktionellen Lipidmembran sind.

8. Verwendung nach Anspruch 6, **dadurch gekennzeichnet, daß** die zweidimensionalen kristallinen Proteinschichten an ihren den sekundären Zellwandpolymeren abgewandten Seiten mit je einer oder mehreren gleichen oder verschiedenen funktionellen Domänen versehen sind.

9. Verwendung nach einem der Ansprüche 1 bis 3, **dadurch gekennzeichnet, daß** die monomolekular gebundenen Moleküle hydrophobe Ketten sind und mit den hydrophilen sekundären Zellwandpolymeren amphiphile Moleküle bilden.

10. Verwendung nach einem der Ansprüche 1 bis 3, **dadurch gekennzeichnet, daß** die Moleküle des Trägers jene von zweidimensionalen kristallinen Proteinschichten sind, wobei die sekundären Zellwandpolymere unter Bildung eines zusammengesetzten Verbundkörpers die Bindeglieder zu einer funktionellen Lipidschicht sind.

11. Verwendung nach Anspruch 10, **dadurch gekennzeichnet, daß** über die funktionelle Lipidschicht zweite zusammengesetzte Verbundkörper spiegelbildlich aneinander gebunden sind.

12. Verwendung nach Anspruch 11, **dadurch gekennzeichnet, daß** die kristallinen Proteinschichten aus gleichen oder unterschiedlichen Proteinmolekülen bestehen.

13. Verwendung nach einem der Ansprüche 10 bis 12, **dadurch gekennzeichnet, daß** die funktionelle Lipidschicht vesikuläre Struktur aufweisen.

14. Verwendung nach Anspruch 1, **dadurch gekennzeichnet, daß** der Träger eine Micelle aus hydrophoben selbstorganisierten Ketten ist.

15. Verbundkörper aus einem Träger und einer Molekülschicht, **dadurch gekennzeichnet, daß** die Moleküle dieser Molekülschicht orientiert monomolekular an dem Träger über extrahierte, gereinigte sekundäre Zellwandpolymerketten gebunden sind, wobei die Enden der Polymerketten modifiziert sind.

16. Verbundkörper nach Anspruch 15, **dadurch gekennzeichnet, daß** die sekundären Zellwandpolymerketten am Träger und/oder den zu bindenden Molekülen über eine lektinartige Bindung verankert sind.

17. Verbundkörper nach Anspruch 15 oder 16, **dadurch gekennzeichnet, daß** auf einer Polymermatrix kristalline Proteinschichten über die sekundären Zellwandpolymere gebunden sind.

18. Verbundkörper nach Anspruch 17, **dadurch gekennzeichnet, daß** an den kristallinen Proteinschichten an der den sekundären Zellwandpolymeren abgewandten Seite Lipidfilme oder -vesikel angeordnet sind.

19. Verbundkörper nach Anspruch 17, **dadurch gekennzeichnet, daß** an den kristallinen Proteinschichten an der den sekundären Zellwandpolymeren abgewandten Seite funktionelle Domänen in regelmäßigen Abständen und Anordnungen vorgesehen sind.

20. Verbundkörper nach Anspruch 19, **dadurch gekennzeichnet, daß** die funktionellen Domänen unterschiedlich, jedoch in regelmäßigen Mustern vorgesehen sind.

21. Verbundkörper nach Anspruch 15 oder 16, **dadurch gekennzeichnet, daß** die sekundären Zellwandpolymere von einer kristallinen Proteinschicht, die vorzugsweise direkt auf einen Träger aufgebracht ist, wegragen und eine funktionelle Lipidschicht binden.

22. Verbundkörper nach Anspruch 21, **dadurch gekennzeichnet, daß** zwei derartige Proteinschichten über die funktionelle Lipidschicht spiegelbildlich gegenüberliegend aneinander gebunden sind.

23. Verbundkörper nach Anspruch 22, **dadurch gekennzeichnet, daß** die eine Proteinschicht aus anderen Proteinmolekülen besteht als die andere Proteinschicht.

24. Verbundkörper nach einem der Ansprüche 15 bis 23, **dadurch gekennzeichnet, daß** er die Mündungsöffnung an einem Ende eines Röhrchens überspannend angeordnet ist.

25. Verbundkörper nach einem der Ansprüche 15 bis 24, **dadurch gekennzeichnet, daß** der Träger eine Micelle aus hydrophoben, selbstorganisierten Ketten ist.

26. Verbundkörper nach einem der Ansprüche 15 bis 25, **dadurch gekennzeichnet, daß** er Träger spezieller funktioneller Moleküle für Diagnostika, Drug-Screening, High Throughputscreening od.dgl ist.

27. Verbundkörper nach einem der Ansprüche 15 bis 25, **dadurch gekennzeichnet, daß** er zur spezifischen Bindung von Zellen, vorzugsweise in Monoschichten, eingesetzt ist wobei gegebenenfalls das SCWP an der Oberfläche der Zellen exponiert ist.

## Claims

1. Use of extracted, purified secondary cell wall polymer from prokaryotic micro-organisms for the oriented monomolecular binding of (functional) molecular layers and/or their attachment to molecules of a support.

2. Use according to claim 1, **characterised in that** the binding of the secondary cell wall polymers is effected via corresponding domains of the functional molecular layers and/or of the support.

3. Use according to claim 1 or 2, **characterised in that** the binding of the secondary cell wall polymers to the molecular layers and/or to the molecules of the support is effected via lectin-like bonds.

4. Use according to one of claims 1 to 3, **characterised in that** the secondary cell wall polymers are the binding members between the polymeric support structures and functional molecules or groups of molecules.

5. Use according to claim 4, **characterised in that** the polymeric support structures are microfiltration membranes.

6. Use according to claim 4 or 5, **characterised in that** the molecular layers are two-dimensional crystalline protein layers.

7. Use according to claim 6, **characterised in that** the two-dimensional crystalline protein layers are in turn supports of a functional lipid membrane.

8. Use according to claim 6, **characterised in that** the two-dimensional crystalline protein layers are each provided with one or more identical or different functional domains, on their sides remote from the secondary cell wall polymers.

9. Use according to one of claims 1 to 3, **characterised in that** the monomolecularly bound molecules are hydrophobic chains and form amphiphilic molecules with the hydrophilic secondary cell wall polymers.

10. Use according to one of claims 1 to 3, **characterised in that** the molecules of the support are molecules of two-dimensional crystalline protein layers, the secondary cell wall polymers being the binding members for a functional lipid layer, to form a composite body.

11. Use according to claim 10, **characterised in that** second composite bodies are bonded to one another in mirror-symmetrical fashion via the functional lipid layer.

12. Use according to claim 11, **characterised in that** the crystalline protein layers consist of identical or different protein molecules.

13. Use according to one of claims 10 to 12, **characterised in that** the functional lipid layer has a vesicular structure.

14. Use according to claim 1, **characterised in that** the support is a micelle of hydrophobic self-organised chains.

15. Composite body comprising a support and a molecular layer, **characterised in that** the molecules of this molecular layer are bound to the support, in a monomolecular orientation, via extracted, purified secondary cell wall polymer chains, the ends of the polymer chains being modified.

16. Composite body according to claim 15, **characterised in that** the secondary cell wall polymer chains are anchored to the support and/or to the molecules which are to be bound via a lectin-like bond.

17. Composite body according to claim 15 or 16, **characterised in that** on a polymer matrix crystalline protein layers are bound via the secondary cell wall polymers.

18. Composite body according to claim 17, **characterised in that** lipid films or vesicles are arranged on the crystalline protein layers on the side remote from the secondary cell wall polymers.

19. Composite body according to claim 17, **characterised in that** functional domains are provided at regular intervals and in regular arrangements on the crystalline protein layers on the side remote from the secondary cell wall polymers.

20. Composite body according to claim 19, **characterised in that** the functional domains are different but are provided in regular patterns.

21. Composite body according to claim 15 or 16, **characterised in that** the secondary cell wall polymers protrude from a crystalline protein layer which is preferably applied directly to a support, and bind a functional lipid layer.

22. Composite body according to claim 21, **characterised in that** two such protein layers are bonded mirror-symmetrically opposite each other via the functional lipid layer.

23. Composite body according to claim 22, **characterised in that** one protein layer consists of different protein molecules from the other protein layer.

24. Composite body according to one of claims 15 to 23, **characterised in that** it is arranged so as to span the opening at one end of a tube.

25. Composite body according to one of claims 15 to 24, **characterised in that** the support is a micelle of hydrophobic, self-organised chains.

26. Composite body according to one of claims 15 to 25, **characterised in that** it is a support of special functional molecules for diagnostics, drug screening, high throughput screening or the like.

27. Composite body according to one of claims 15 to 25, **characterised in that** it is used for the specific binding of cells, preferably in monolayers, the SCWP optionally being exposed on the surface of the cells.

## Revendications

1. Utilisation de polymères membranaires secondaires extraits, purifiés, de microorganismes procaryotes, pour la liaison orientée monomoléculaire de couches (fonctionnelles) de molécules et/ou leur fixation sur les molécules d'un support.

2. Utilisation selon la revendication 1, **caractérisée en ce que** la liaison des polymères membranaires secondaires est réalisée par des domaines appropriés des couches fonctionnelles de molécule et/ou du support.

3. Utilisation selon la revendication 1 ou 2, **caractérisée en ce que** la liaison des polymères membranaires secondaires est réalisée, sur les couches de molécule et/ou les molécules du support par des liaisons de type lectine.

4. Utilisation selon l'une quelconque des revendications 1 à 3, **caractérisée en ce que** les polymères membranaires secondaires sont les maillons de liaison entre les structures polymères du support et les molécules ou des groupes de molécules fonctionnelles.

5. Utilisation selon la revendication 4, **caractérisée en ce que** les structures polymères du support sont des membranes de microfiltration.

6. Utilisation selon la revendication 4 ou 5, **caractérisée en ce que** les couches de molécules sont des couches bidimensionnelles de protéines cristallines.

7. Utilisation selon la revendication 6, **caractérisée en ce que** les couches bidimensionnelles de protéines cristallines sont pour leur part, le support d'une membrane lipidique fonctionnelle.

8. Utilisation selon la revendication 6, **caractérisée en ce que** les couches bidimensionnelles de protéines cristallines sont munies, sur leur face opposée aux polymères membranaires secondaires, de un ou plusieurs domaines fonctionnels identiques ou différents.

9. Utilisation selon l'une quelconque des revendications 1 à 3, **caractérisée en ce que** les molécules liées de manière monomoléculaire sont des chaînes hydrophobes et forment avec les polymères membranaires secondaires hydrophiles, des molécules amphiphiles.

10. Utilisation selon l'une quelconque des revendications 1 à 3, **caractérisée en ce que** les molécules du support sont celles des couches bidimensionnelles de protéines cristallines, où les polymères membranaires secondaires sont les maillons de liaison à une couche lipidique fonctionnelle pour former un corps de liaison assemblé.

11. Utilisation selon la revendication 10, **caractérisée en ce que** des deuxièmes corps de liaison assemblés sont reliés de manière inverse par la couche lipidique fonctionnelle.

12. Utilisation selon la revendication 11, **caractérisée en ce que** les couches de protéines cristallines consistent en des molécules de protéines identiques ou différentes.

13. Utilisation selon l'une quelconque des revendications 10 à 12, **caractérisée en ce que** la couche lipidique fonctionnelle présente une structure vésiculaire.

14. Utilisation selon la revendication 1, **caractérisée en ce que** le support est un micelle de chaînes hydrophobes auto-organisées.

15. Corps de liaison, composé d'un support et d'une couche moléculaire, **caractérisé en ce que** les molécules de cette couche moléculaire sont liées de manière orientée et monomoléculaire, sur le support par des chaînes de polymère membranaire secondaire extrait, purifié, où les extrémités des chaînes de polymères sont modifiées.

16. Corps de liaison selon la revendication 15, **caractérisé en ce que** les chaînes de polymères membranaires secondaires sont ancrées sur le support et/ou les molécules à lier par une liaison de type lectine.

17. Corps de liaison selon la revendication 15 ou 16, **caractérisé en ce que** les couches de protéines cristallines sont liées par les polymères membranaires secondaires sur une matrice polymère.

18. Corps de liaison selon la revendication 17, **caractérisé en ce que** des films ou vésicules lipidiques sont disposés sur les couches de protéines cristallines, sur la face opposée aux polymères membranaires secondaires.

19. Corps de liaison selon la revendication 17, **caractérisé en ce que** des domaines fonctionnels sont prévus sur les couches de protéines cristallines, sur la face opposée aux polymères membranaires secondaires, à intervalle et selon un arrangement réguliers.

20. Corps de liaison selon la revendication 19, **caractérisé en ce que** des domaines fonctionnels différents sont prévus, mais selon un schéma régulier.

21. Corps de liaison selon la revendication 15 ou 16, **caractérisé en ce que** les polymères membranaires secondaires dépassent d'une couche de protéines cristallines, qui est de préférence, directement appliquée sur le support et lient une couche lipidique fonctionnelle.

22. Corps de liaison selon la revendication 21, **caractérisé en ce que** deux couches de protéines de ce type sont liées par la couche lipidique fonctionnelle, opposées, inversées.

23. Corps de liaison selon la revendication 22, **caractérisé en ce que** l'une des couches de protéines consiste en d'autres molécules de protéine que l'autre couche de protéines.

24. Corps de liaison selon l'une quelconque des revendication 15 à 23, **caractérisé en ce qu'**il est disposé en recouvrant l'embouchure de l'extrémité d'un tube.

25. Corps de liaison selon l'une quelconque des revendication 15 à 24, **caractérisé en ce que** le support est un micelle de chaînes hydrophobes, auto-organisées.

26. Corps de liaison selon l'une quelconque des revendication 15 à 25, **caractérisé en ce qu'**il est le support de molécules fonctionnelles spéciales pour le diagnostic, le criblage de médicaments, un criblage à haut débit, ou similaire.

27. Corps de liaison selon l'une quelconque des revendication 15 à 25, **caractérisé en ce qu'**il est mis en oeuvre pour la liaison spécifique de cellules, de préférence en monocouches, où le cas échéant, le SCWP est exposé sur la surface des cellules.
